# EUROPEAN PATENT SPECIFICATION

(11) **EP 4 051 339 B1**
(45) Date of publication and mention of the grant of the patent: **10.12.2025**
(21) Application number: 20796747.2
(22) Date of filing: 21.10.2020
(51) Int. Cl.: A61M 5/162, A61M 5/142, A61M 5/145

(54) **AN ASSEMBLY FOR A CARTRIDGE NEEDLE INSERTION MECHANISM**
ANORDNUNG FÜR EINEN KARTUSCHENNADELEINFÜHRMECHANISMUS
ENSEMBLE POUR UN MÉCANISME D'INSERTION DE CARTOUCHE-AIGUILLE

(30) Priority: 31.10.2019 EP 19206382
(43) Date of publication of application: 07.09.2022
(73) Proprietor: Ypsomed AG, 3401 Burgdorf (CH)
(72) Inventor: BURREN, Stefan, 3150 Schwarzenburg (CH); BERNHARD, Mario, 3400 Burgdorf (CH); MELLENBERGER, Andres, 3425 Koppigen (CH); TSCHIRREN, Markus, 3400 Burgdorf (CH); SCHENKER, Susanne, 4912 Aarwangen (CH); SCHRUL, Christian, 3400 Burgdorf (CH)
(74) Representative: Meier Obertüfer, Jürg
(86) International application number: PCT/EP2020/079537
(87) International publication number: WO 2021/083746

(56) References cited:
- EP-A1- 3 260 149
- EP-A2- 3 348 284
- WO-A1-2017/089286
- US-A1- 2017 100 305

## Description

### FIELD OF THE INVENTION

The current invention relates to an assembly for a cartridge needle insertion mechanism comprising a cartridge needle slider that moves a cartridge needle holder holding a needle such that a septum of a cartridge is penetrated by the needle. A compliant coupling between the cartridge needle holder and the cartridge slider reduces the risk of leakage when the needle is inserted into the cartridge.

### BACKGROUND OF THE INVENTION

Fluid medicaments are delivered to patients from a reservoir containing the medicament either via intravenous or subcutaneous injection. A piston or plunger in the reservoir is advanced by a delivery mechanism towards an outlet of the reservoir such that medicament located between the piston and the outlet can be injected via a needle that is inserted into the patient. The reservoir selected is often a cartridge such as a glass cartridge which is well accepted in the pharmaceutical industry as a primary packaging material. The cartridge comprises a glass barrel enclosing the medicament between the piston on one side and a septum on the opposite side. For delivery, a fluid conduit must be connected to the medicament in the cartridge on one end and a skin needle on the other end. The connection to the medicament in the cartridge may be established by either piercing the piston or the septum with a cartridge needle. The fluid connection to the cartridge may be established just before injection, e.g. the fluid conduit is not connected to the cartridge during shelf life of the product, or alternatively, the fluid connection is present throughout the lifecycle of the product.

The fluid medicaments may be delivered to patients using an injection or infusion device comprising the delivery mechanism, the cartridge and the fluid conduit comprising the skin needle and the cartridge needle. Additionally, the injection or infusion device may have a needle insertion mechanism for automatic insertion of the skin needle into the skin of the patient. The other end of the fluid conduit with the cartridge needle may be connected to the cartridge just before use having the advantage that there is less risk of contamination of the medicament by components of the fluid conduit (leachables) as there is only contact for a limited time period during injection. Additionally, the fill-finish of the product is separate from the assembly with the fluid conduit. Thus the cartridge may be filled in a standardized sterile environment and the assembly of the device with the filled cartridge and the delivery mechanism including the fluid conduit may be done under non-sterile clean room conditions making the assembly easier.

If the cartridge needle (connected to the conduit) is inserted into the cartridge just before using the injection or infusion device, then the cartridge may already be present in a so-called "ready-to-use" device or, alternatively, the user inserts the cartridge into an empty cartridge holder of the device and establishes the connection between the cartridge and the cartridge needle, for example, by closing the device. In some applications, relative expensive medicaments are delivered by the device and a "ready-to-use" device is preferred by the pharmaceutical industry since it increases the reliability of the therapy and prevents loss of medicament due to handling failures during cartridge insertion into the holder or cartridge fracture.

In "ready-to-use" devices, the connection between the cartridge needle and the cartridge may be established by either moving the cartridge in a cartridge holder versus a fixed cartridge needle thereby penetrating the septum (or piston) of the cartridge. The cartridge may be moved in the cartridge holder towards the needle using a cartridge insertion mechanism that automatically advances the cartridge (for example by a spring) or the user manually advances the cartridge in the holder. Alternatively, the cartridge needle is moved by a cartridge needle insertion mechanism that automatically inserts the cartridge needle in the septum of a cartridge that is axially fixed in the cartridge holder.

An example of a "ready-to-use" device is a bolus or patch injector as presented in EP3067083B1 where a cartridge is moved towards a collar comprising the cartridge needle to establish a fluid connection. In EP3539592A1, an example is shown for a "ready-to-use" device where a cartridge needle is moved towards a cartridge that is axially fixed in the cartridge holder.

A problem of devices where the fluid connection is established just prior to use (e.g. no connection during shelf-life) is that the needle may penetrate the septum under an angle that is not perpendicular to the septum. As the sharp needle tip cuts through the septum the penetration hole may be enlarged and off-axis needle penetration forces may act on the needle such that the liquid medicament may leak through the interface between the cartridge needle and the septum. This may waist expensive medicament and lead to under dosing as part of the medicament will not flow through the fluid conduit into the patient.

For devices where the cartridge is moved versus a fixed cartridge needle, e.g. either "ready-to use" or where the patient moves the cartridge in the cartridge holder. The off-axis insertion is enhanced by the movement of the cartridge with respect to a cartridge holder. Especially the glass cartridges have relative low dimensional tolerances inherently linked to material and manufacturing method used. The movement of the cartridge versus a cartridge holder (often made from materials and methods having higher dimensional tolerances) results in misalignment of the cartridge with respect to the needle. In EP3067083B1, this problem is solved by a compliant support that aligns the collar comprising the cartridge needle with the cartridge. The cartridge is moved with respect to the collar and the collar is aligned with the cartridge as the collar engages the cartridge. Any misalignment of the cartridge needle with respect to the collar cannot be compensated and this aspect still may lead to leakage. In EP3539592A1, the cartridge needle is moved by a cartridge needle slider towards the fixed cartridge. Any off-axis insertion of the cartridge needle is not compensated for, potentially leading to leakage and loss of medicament.

It is an objective to provide an assembly for a cartridge needle insertion mechanism where a cartridge needle is moved towards, and inserted into a cartridge and reduce the leakage risk cartridge due to off-axis septum penetration.

This objective is solved by the present invention by a compliant coupling present between a cartridge needle holder holding the cartridge needle and a cartridge needle slider. A driver moves the cartridge needle slider together with the holder and the needle towards the cartridge. The compliant coupling between slider and holder is configured to facilitate movement of the cartridge needle holder (with the needle) with respect to the cartridge needle slider when moved into the second position thereby compensating for needle penetration forces that are directed perpendicular to the insertion direction.

### DEFINITIONS

In the present context, the terms "substance", "drug", "medicament" and "medication" are to be understood to include any flowable medical formulation suitable for controlled administration through means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an infusion set, an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a comprising the cartridge needle with the cartridge. The cartridge is moved with respect to the collar and the collar is aligned with the cartridge as the collar engages the cartridge. Any misalignment of the cartridge needle with respect to the collar cannot be compensated and this aspect still may lead to leakage. In EP3539592A1, the cartridge needle is moved by a cartridge needle slider towards the fixed cartridge. Any off-axis insertion of the cartridge needle is not compensated for, potentially leading to leakage and loss of medicament.

EP3260149 Al and EP3348284A1 disclose an injection device with a fluid path comprising a spike attached to a spike inserter carrier. The spike inserter carrier is configured to move the spike through a septum of a cartridge.

It is an objective to provide an assembly for a cartridge needle insertion mechanism where a cartridge needle is moved towards, and inserted into a cartridge and reduce the leakage risk cartridge due to off-axis septum penetration.

The invention is defined by the subject-matter of claim 1.

Further embodiments are defined in the dependent claims.

This objective is solved by the present invention by a compliant coupling present between a cartridge needle holder holding the cartridge needle and a cartridge needle slider. A driver moves the cartridge needle slider together with the holder and the needle towards the cartridge. The compliant coupling between slider and holder is configured to facilitate movement of the cartridge needle holder (with the needle) with respect to the cartridge needle slider when moved into the second position thereby compensating for needle penetration forces that are directed perpendicular to the insertion direction. DEFINITIONS

In the present context, the terms "substance", "drug", "medicament" and "medication" are to be understood to include any flowable medical formulation suitable for controlled administration through means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an infusion set, an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours. An "on body delivery system" where a certain volume, a "bolus" is injected, is an injection system. An "on body delivery system" where a medicament is delivered at a certain delivery rate, for example a basal rate, , is an infusion system.

In the claims, the word "comprising" does not exclude other elements or steps, and the indefinite article "a" or "an" does not exclude a plurality. For example "a first coupling" does not exclude the fact that there may be two "first coupling members" that functionally or structurally fulfill the purpose of "a first coupling member". The mere fact that certain elements or steps are recited in distinct claims shall not preclude the existence of further meaningful combinations of these elements or steps.

The term "penetration forces" are forces acting on the insertion needle when or during insertion of the needle along an insertion axis into a septum. The forces may be oriented parallel to an insertion direction defining an insertion axis for the insertion needle, or the forces, or a resultant component of the forces may be directed perpendicular to the insertion axis. The latter are may also be called "off-axis" penetration forces. The penetration forces act on the needle or on a part directly coupled thereto, for example a needle holder, or alternatively the penetration forces act on a part indirectly coupled to the needle, for example a cartridge needle slider or a housing.

### DESCRIPTION OF THE INVENTION

In an embodiment according to the invention an assembly for a cartridge needle insertion mechanism is presented. The assembly comprises a housing, which may be the main housing or a housing part connected to the main housing, a cartridge needle defining a cartridge needle axis and a cartridge closed by a septum. The cartridge may be a glass cartridge or made from a plastic material such as a cycloolefinic copolymer (COC). Furthermore the assembly comprises a cartridge needle slider which is linearly guided by the housing along an insertion axis such that the slider can be moved by a driver from a first position, where the cartridge needle is not penetrating the septum, to a second position where the cartridge needle penetrates the septum of the cartridge.

A cartridge needle holder is holding the cartridge needle and the cartridge needle holder is operatively coupled with the cartridge needle slider to be moved together with the cartridge needle slider along the insertion axis from the first to the second position. As the cartridge needle slider is moved by the driver towards the second position, the cartridge needle holder with the needle is slaved towards the second position as well. The assembly is characterized by a compliant coupling that is structurally or functionally located between the cartridge needle holder and the cartridge needle slider. The compliant coupling is resilient and capable to absorb, reduce or counteract forces directed to the coupling or to coupling members forming the compliant coupling. The compliant coupling is configured to facilitate movement of the cartridge needle holder with respect to the cartridge needle slider when moved into the second position, thereby at least partially compensating, reducing or absorbing needle penetration forces. Preferably, the compliant coupling compensates, reduces or absorbs needle penetration forces oriented perpendicular to the insertion axis.

The cartridge needle moves from the first to the second position and the needle with its needle axis may enter the septum off-axis or oblique to the insertion axis. Reasons for the off-axis entrance may be due to the fast movement (moment of inertia) of the cartridge needle, or due to dimensional tolerances of the linear guidance of the cartridge needle slider or because of an off-axis inserted /installed needle in the cartridge needle holder. This may have several effects: i) the tip of the needle cuts through the septum thereby enlarging the entrance hole for the needle ii) the needle may bend during penetration, giving rise to elastic forces acting on the needle which may be oriented perpendicular to the insertion axis, iii) forces may act from the elastic septum onto the needle when the needle has penetrated the septum at an angle deviating from, e.g. not being parallel to, or coincidence with, the insertion axis. These forces may be directed along the insertion axis, or may be partially directed perpendicular to the insertion axis. If not compensated for, or reduced, then the forces, or force components acting perpendicular to the insertion axis (effects ii and iii) and/or the enlarged entrance hole (i), may promote leakage of medicament from the cartridge to the ambient along the interface between the needle and the septum. The insertion forces directed perpendicular operate from the septum to the needle and subsequently to the needle holder such that the complaint coupling between the cartridge needle holder and the cartridge needle slider is deformed thereby absorbing, reducing or compensating these forces and the cartridge needle holder may be displaced with respect to the cartridge needle slider. The cartridge needle slider is linearly guided by the housing along the insertion axis, the guidance being perpendicular to the off-axis forces acting on the needle and therewith these forces are finally guided to the housing. The compliant coupling therewith reduces or eliminates the risk of leakage and improves the reliability of the device comprising the cartridge needle insertion mechanism.

The compliant coupling is configured to facilitate movement of the cartridge needle holder with respect to the cartridge needle slider when moved into the second position. This movement of the cartridge needle holder with respect to the cartridge needle slider is perpendicular to the insertion axis, or the movement is a pivoting movement of the cartridge needle holder around the insertion axis or the cartridge needle holder rotates around the insertion axis.

In the example mentioned above, at least two sources, ii) and iii) are mentioned resulting in a force acting on the needle when inserted at an angle deviating from the insertion axis and which result in a force, or a force component acting on the needle and oriented perpendicular to the insertion axis. Alternatively and additionally, also torque can be exerted onto the needle which may be compensated for by a pivoting or rotational movement of the cartridge needle holder versus the cartridge needle slider and the compliant coupling may compensate those forces as well.

The compliant coupling comprises a first coupling member on the cartridge needle holder engaging a second coupling member on the cartridge needle slider in a plane parallel to the insertion axis.

The first coupling member on the cartridge needle holder engages the second coupling member on the cartridge needle slider in a plane parallel to the needle axis such that the needle penetration forces in the perpendicular direction may deform the compliant coupling consisting of the first and second coupling members. The forces in the perpendicular direction are directed to a plane parallel to the needle axis and via the linear guidance of the cartridge needle slider effectively guided to the housing to reduce the risk of leakage. The first coupling member may comprise a plurality of first coupling members to compensate for needle penetration forces directed in several directions that are all perpendicular to the insertion axis. The first coupling members may be circumferentially arranged around the cartridge needle holder engaging a plurality of second coupling members arranged on the cartridge needle slider. The cartridge needle holder is preferably arranged inside the cartridge needle slider, thus a plurality of first coupling members may radially extend outwards from the cartridge needle holder engaging a plurality of second coupling members that may extend radially inwards from the cartridge needle slider.

In advantageous embodiments, the first coupling member and the second coupling member elastically engage each other and/or are in a linear or rotational friction fit engagement. When the two coupling members engage each other in a plane parallel to the needle axis then the elastic or frictional forces of the engagement are configured to compensate the needle penetration forces when the compliant coupling is deformed by moving the first coupling member relative to the second coupling member or vice versa moving the second coupling member relative to the first coupling member. When the first and second coupling members are moved relative to another then the position of the cartridge needle holder may change with respect to the cartridge needle slider as the compliant coupling is deformed. Therewith also the position of the part of the cartridge needle that is coupled to the cartridge needle holder may change during insertion. The engagement between the cartridge needle holder and the cartridge needle slider, via at least one pair of coupling members, is an elastic engagement, a frictional engagement or a combination thereof. Optionally one or both of the coupling members may be plastically deformed after the elastic deformation. The change of position between the cartridge needle holder and the cartridge needle slider may be a movement perpendicular to the insertion axis, or the cartridge needle holder may rotate or tilt relative to the cartridge needle slider. Thus there are more options available for shaping and designing the compliant coupling by combining frictional, elastic or rotational force compensators. The relative movement between the cartridge needle holder with respect to the slider may trigger a signal, for example an acoustic signal.

When the cartridge needle axis deviates too much from the insertion axis, this may be due to a failure and the acoustic signal may be a warning signal. Alternatively the relative movement between the slider and the holder is followed by a sensor or an electrical contact is established providing a visual (LED) or acoustic (vibra alarm) to the user that the device is, due to a bend needle, out of specification and a health care professional should be contacted

In further embodiments the first coupling member comprises at least one protrusion radially extending from the cartridge needle holder whereby the protrusion is at least partially elastically and/or plastically deformable and engages at least one second coupling member shaped as a rigid surface on the cartridge needle slider. The second coupling member is oriented parallel to the insertion axis. Alternatively at least one first coupling member comprises a rigid protrusion radially extending from the cartridge needle holder engaging at least one second coupling member shaped as an at least partially elastically and/or plastically deformable surface on the cartridge needle slider. The second coupling member again being oriented parallel to the insertion axis in the alternative embodiment. A protrusion radially extending from the cartridge needle holder is not limited to a single protrusion but defines at least one protrusion. Preferably there are two protrusions, more preferably three protrusions, most preferably four protrusions. The second coupling member is not restricted to a single second coupling member but engages each of the first coupling members. Therefore there are preferably two second coupling members, more preferably three second coupling members and most preferably four second coupling members. The coupling members form pairs and ensure that the cartridge needle holder is held in a stable position with respect to the cartridge needle slider when in the first position. During movement of the cartridge needle slider from the first towards the second position (when the cartridge needle has not penetrated yet the septum) then the at least one pair of coupling members ensure that the cartridge needle holder remains in a stable position. Upon penetration of the septum, penetration forces perpendicular to the insertion axis and acting on the insertion needle may occur and then the first and second coupling members of at least one pair of coupling members are moved relative to another such that the position of the cartridge needle holder shifts with respect to the cartridge needle slider as the compliant coupling is deformed. The relative movement of the first and second coupling members of at least one pair of coupling members results in the above mentioned elastic deformation, friction or plastic deformation thereby compensating, absorbing or reducing the penetration forces perpendicular to the insertion axis.

The cartridge comprises a barrel, optionally a glass barrel, defining a cartridge axis and the cartridge is in a fixed position with respect to the housing and wherein the cartridge axis is aligned with the insertion axis when the cartridge needle slider is in the first position. The cartridge is in a fixed position with respect to the housing and the device is a prefilled device ("ready-to-use"), e.g. the cartridge is already in the device and is ready to use such that the patient does not have to insert a cartridge in the device. Moving the cartridge in a device may lead to larger off-axis movements due to the relatively large dimensional tolerances of (glass) cartridges and movement over a longer distance, e.g. at least the length of the cartridge. By using a prefilled device, the alignment of the cartridge and the cartridge needle holder is not critical compared to a device requiring movement of the cartridge. In a prefilled device, the cartridge needle slider has to move over a shorter distance for needle insertion and the parts of the slider, the coupling members and the housing are made with high precision and low dimensional tolerances. Therefore the cartridge needle slider can be precisely aligned with the cartridge (and the septum). The alignment of the cartridge axis and the needle axis is an advantage for penetrating the septum in the septum's center such that the cartridge needle enters the center of the neck of the of the cartridge's barrel when penetrating the septum to establish a fluid connection between the medicament in the cartridge and the cartridge needle.

In advantageous embodiments, the assembly is not an alignment arrangement configured to align the cartridge axis and the insertion axis when the cartridge needle slider moves from the first position to the second position. An off axis needle penetration, e.g. the needle axis being oblique to the insertion axis, may be allowed in the present invention resulting in needle penetration forces partially compensated for by a shift of the cartridge needle holder with respect to the cartridge needle slider but not fully aligning the needle axis to the insertion axis. Thus after insertion through the septum the needle axis may still be oblique to the insertion axis. The assembly may advantageously used in combination with prefilled devices or devices without moving the cartridge with respect to a fixed insertion needle.

In advantageous embodiments, the cartridge needle slider and the cartridge needle holder are coaxially arranged around the insertion axis when the cartridge needle slider is in the first position. The cartridge needle slider is inserted into the cartridge needle holder and the coaxial arrangement is beneficial for alignment of the parts during assembly.

The cartridge needle holder may be moved out of the coaxial arrangement with respect to the cartridge needle slider when the needle axis is not parallel to the insertion axis when the cartridge needle slider is moved into the second position thereby generating needle penetration forces perpendicular to the insertion axis deforming the compliant coupling. If the cartridge needle axis and the cartridge axis remain aligned during movement of the cartridge needle slider from the first to the second position then the septum will be penetrated perpendicular to, and in the center of the septum such that no off-axis penetration forces are generated. In both cases, needle penetration forces acting on the needle and directed along the insertion axis will occur, but those will not be compensated for, absorbed or reduced by the compliant coupling.

The cartridge needle holder is axially fixed to the cartridge needle slider but allowing movement with respect to the cartridge needle holder perpendicular to the insertion axis, or a pivoting or a rotational movement around the insertion axis against a bias or resistance of the compliant coupling. The cartridge needle slider is linearly guided by the housing by a splined engagement defining the insertion axis.

The cartridge needle holder is axially fixed when inserted into the cartridge needle slider to transfer the linear movement of the cartridge needle slider to the cartridge needle holder. The axial fixation also ensures that the needle penetration forces directed parallel to the insertion axis are compensated for or absorbed. Optionally a gearing mechanism, a motion link or a threaded engagement transfers the linear motion of the cartridge needle slider to the cartridge needle holder with a defined transmission ratio. Preferably, the cartridge needle holder is axially fixed, preferably by stop surfaces on the cartridge needle slider and counter stop surface on the cartridge needle holder. When the cartridge needle slider is moved from the first to the second position the stop surface and counter stop surface are in abutment.

In some embodiments, the cartridge needle in the assembly is connected to the cartridge needle holder by an elastic member preferably being an elastic glue.

The cartridge needle slider comprising the cartridge needle holder and the needle are moved from the first to the second position by a driver, preferably a spring for an efficient and fast insertion, preferably within milliseconds. The forces acting on the needle during penetration which are oriented parallel to the insertion axis are guided to the cartridge needle holder and may damage the connection between them and therefore an elastic glue is beneficial as it introduces an elastic member functioning as a shock absorber for the connection and thereby reduces the fracture risk for the connection due to needle penetration forces oriented along the insertion axis. The fracture risk may be higher if a rigid glue like a thermosetting epoxy or an acrylic based glue is used. Preferably a silicone based glue is used, preferably a medical grade adhesive according to USP Class VI or ISO 10993-5.

The driver in the assembly may be a spring located between the housing and the cartridge needle slider and acting on the housing and the cartridge needle slider, the spring being retained in a compressed state to provide a biasing force on the housing and the cartridge needle slider when the cartridge needle slider is held in the first position with respect to the housing by a releasable locking feature.

The cartridge needle insertion may be spring driven such that the user must not manually provide the force to penetrate the septum, additionally, a spring ensures that the septum can be penetrated at a high speed such that the septum is penetrated within milliseconds, preferably below 10 milliseconds, more preferably below 5 milliseconds. This in contrast to devices that are not prefilled, or where a cartridge is inserted manually and during insertion also the septum is penetrated by a cartridge needle that is fixed with respect to the housing. A knob on the cartridge needle slider may engage a locking feature, preferably shaped as a locking fork on a slider and the slider (e.g. a slider different from the cartridge needle slider) can be moved from a starting position where the locking fork engages the knob on the cartridge needle slider such that the cartridge needle slider is held in the first position. The slider is linearly guided by the housing (splined for example) and when the slider is moved, the engagement between the locking fork and the knob is released such that the spring decompresses and moves the cartridge needle slider from the first to the second position. Preferably the linear guidance of the slider with respect to the housing and the linear guidance of the cartridge needle slider with respect to the housing are oriented perpendicular to another. The spring may be selected from a compression spring, a spiral spring, a cone-shaped spiral spring, a torsional spring or a leaf spring.

The first coupling member may be hollow and may be part of, or form a passage for, a fluid conduit establishing a fluid link between a medicament present in the cartridge and the cartridge needle.

The fluid conduit comprises several parts, such as a flexible tube connecting the cartridge needle holder holding the cartridge needle on one end, and a skin needle holder holding a skin needle on the other end. The fluid conduit must be guided when the cartridge needle holder moves to the second position and/or when the skin needle is inserted into the patient's surface. Integrating the one of the first coupling members into the fluid conduit may be beneficial for fixating the fluid conduit (or tube) to the cartridge needle slider. This may reduce the number of parts required to fixate the fluid conduit into the device as the cartridge needle holder may be snap fitted into the cartridge needle slider. For that purpose one of the first coupling members may be hollow allowing for fluid transport from the cartridge through the cartridge needle and via the cartridge needle holder having the hollow coupling member to the tubing and finally via the skin needle into the patient.

The cartridge needle is a hollow needle whereby the tip of the needle is sharpened and may have a closed end such as a pencil tip needle or the tip of the needle is sharpened and may have an open end.

The tip of the needle is sharpened to facilitate the penetration of the needle through the septum as the cartridge needle slider moves from the first to the second position. The pencil tip needle has a closed end at the tip and at least one opening in a side wall of the needle. As the pencil tip needle penetrates the septum of the cartridge, the sharp tip penetrates the septum without the risk of punching the septum resulting in coring and/or fragmentation (particles) of the septum potentially leading to needle clogging. A hollow tip of the needle is also sharpened and may punch the septum such that the end of the hollow needle may get blocked. The use of a pencil tip needle may increase the reliability of the device. The use of a compliant coupling in the present invention may reduce the risk of punching the septum and thereby decreasing the risk of needle blocking if a hollow (or open) needle tip is used.

The cartridge needle holder may be snap-fitted into the cartridge needle slider. The snap fit connection facilitates the assembly process of the cartridge needle holder into the cartridge needle slider. The snap fit connection may be formed by flexible arms, deformable hooks and other snap fitting means. Preferably the snap fit connectors are made from plastic and an integral part of the cartridge needle slider or the cartridge needle holder and made using injection molding of a plastic. Preferably the cartridge needle slider is made from a stiff material such as a glass fiber reinforced polyamide whereas the cartridge needle holder is preferably made from a polyester such as PBT or a medical grade cyclo olefinic copolymer (COC). Alternatively, the snap fit connection is established using a third part such as a stamped metal part. Preferably, the snap fit connection is not releasable.

A further embodiment comprises an injection device with the assembly for the cartridge needle insertion mentioned above and wherein the injection device is a prefilled patch injection device comprising a skin adhesive surface and a skin needle configured to move from a needle retracted position in the housing to a needle inserted position at least partially outside of the housing after the cartridge needle slider has moved from the first position to the second position.

### BRIEF DESCRIPTION OF THE DRAWINGS

The subject matter of the invention will be explained in more detail in the following text with reference to preferred exemplary embodiments which are illustrated in the attached drawings, in which:
Figure 1: a perspective view of a needle insertion and retraction module including a needle insertion and retraction mechanism,
Figure 2: a cross-sectional view particularly through a needle carrier,
Figure 3: a control element which is engaged with the needle carrier,
Figure 4: the control element disengaging the needle carrier,
Figure 5: a first intermediate member between a spring arm and a needle carrier with the needle carrier in a needle retracted position,
Figure 6: the parts of Figure 5 with the needle carrier in a needle insertion position,
Figure 7: a further view of the needle carrier in a needle insertion position,
Figure 8: a spike carrier with a spike in a first position,
Figure 9: a cross-sectional view of the spike carrier in a first position,
Figure 10: a cross-sectional view of the spike carrier being released to be subsequently moved in a second position,
Figure 11: a cross-sectional view of the spike carrier in a second position,
Figure 12: the first intermediate member disengaged from the needle carrier,
Figure 13: a second intermediate member between a second spring arm and the needle carrier, wherein the intermediate member is disengaged from the housing and engaged with the needle carrier,
Figure 14: the needle carrier which has been moved in the retracted position,
Figure 15: a perspective view of the needle carrier which has been moved in the retracted position,
Figure 16: a control element in its starting position,
Figure 17: a control element which has been moved in the needle release position,
Figure 18: the control element which has been moved in its needle retraction position,
Figure 19: a spring for moving the needle carrier in the needle insertion direction and in the needle retraction direction,
Figures 20 a to c: Cartridge needle insertion with cartridge needle axis parallel to the insertion axis and the forces in the insertion direction (N) and perpendicular to the insertion direction (Q). Needle tip penetrates top surface septum (Figure 20a); Needle tip penetrates through the septum (Figure 20b); Needle fully penetrates through the septum (Figure 20c),

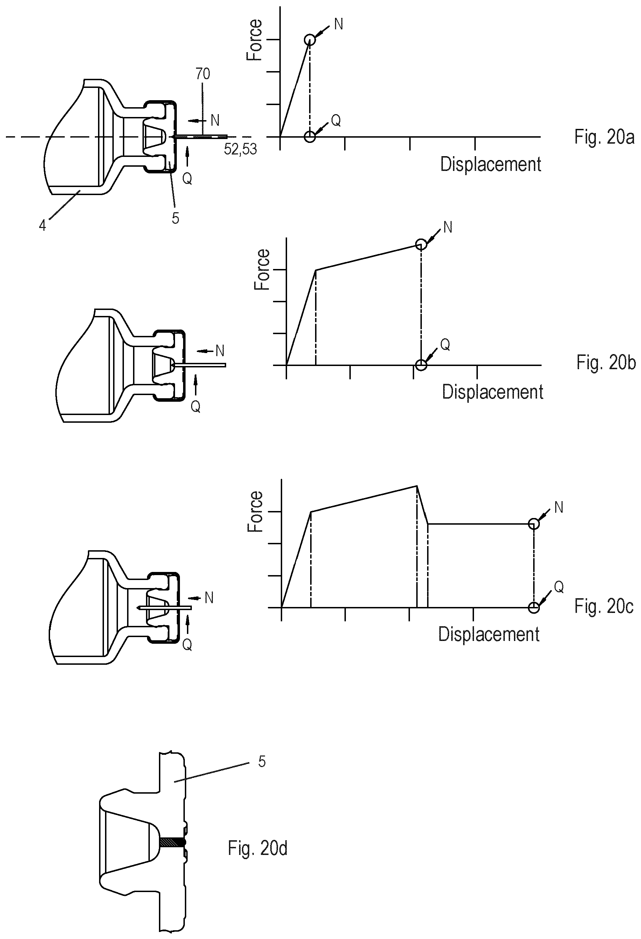

Figure 20 d: Cross section of septum when cartridge needle insertion with cartridge needle axis parallel to the insertion axis,

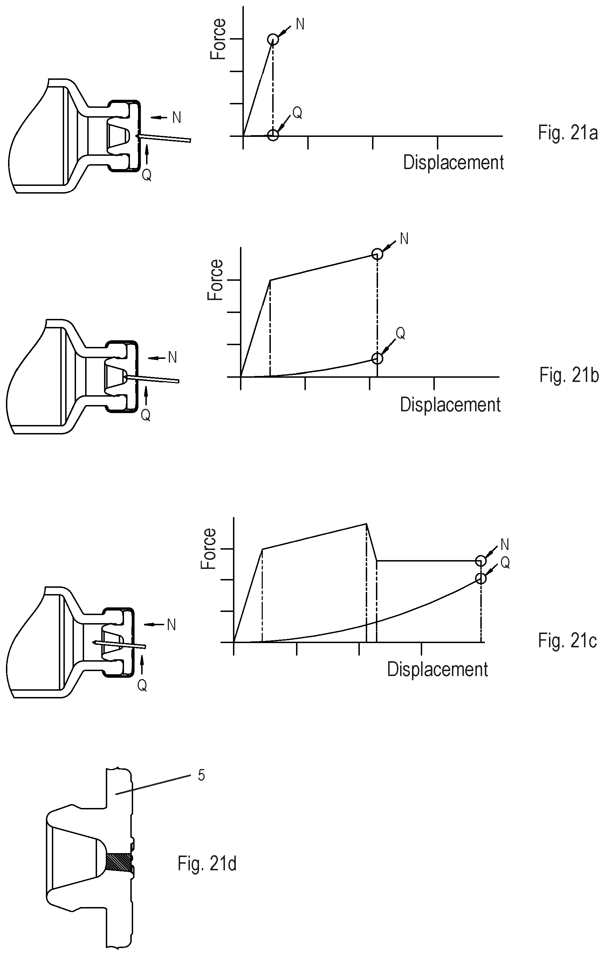

Figures 21 a to c: Cartridge needle insertion with cartridge needle axis oblique to the insertion axis and the forces in the insertion direction (N) and perpendicular to the insertion direction (Q). Needle tip penetrates top surface septum (Figure 21a); Needle tip penetrates through the septum (Figure 21b); Needle fully penetrates through the septum (Figure 21c),
Figure 21 d: Cross section of septum when cartridge needle insertion with cartridge needle axis oblique to the insertion axis without a compliant coupling,

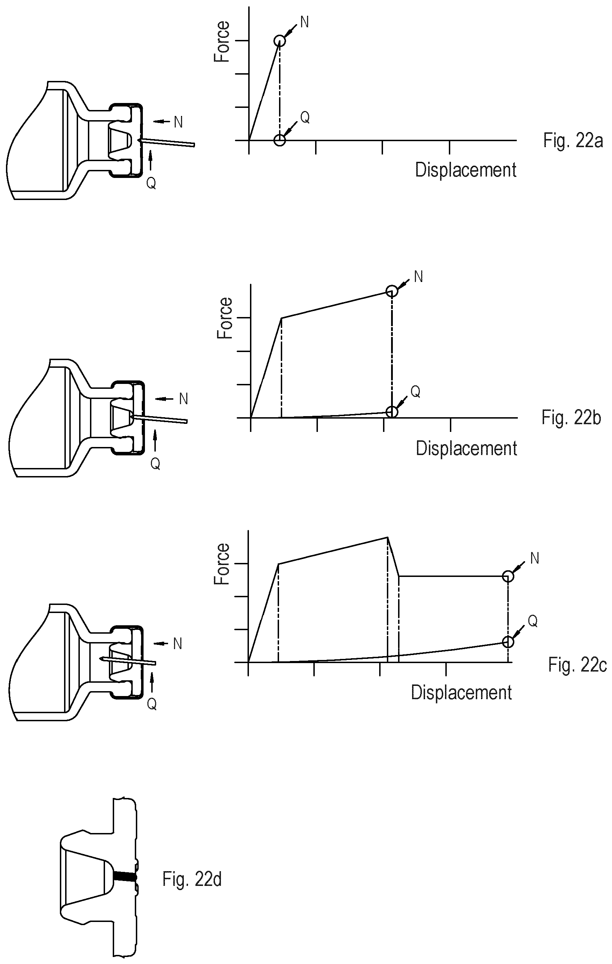

Figures 22 a to c: Cartridge needle insertion with a compliant coupling mechanism and cartridge needle axis oblique to the insertion axis and the forces in the insertion direction (N) and perpendicular to the insertion direction (Q). Needle tip penetrates top surface septum (Figure 22a); Needle tip penetrates through the septum (Figure 22b); Needle fully penetrates through the septum (Figure 22c),
Figure 22 d: Cross section of septum when cartridge needle insertion with cartridge needle axis oblique to the insertion axis with a compliant coupling,

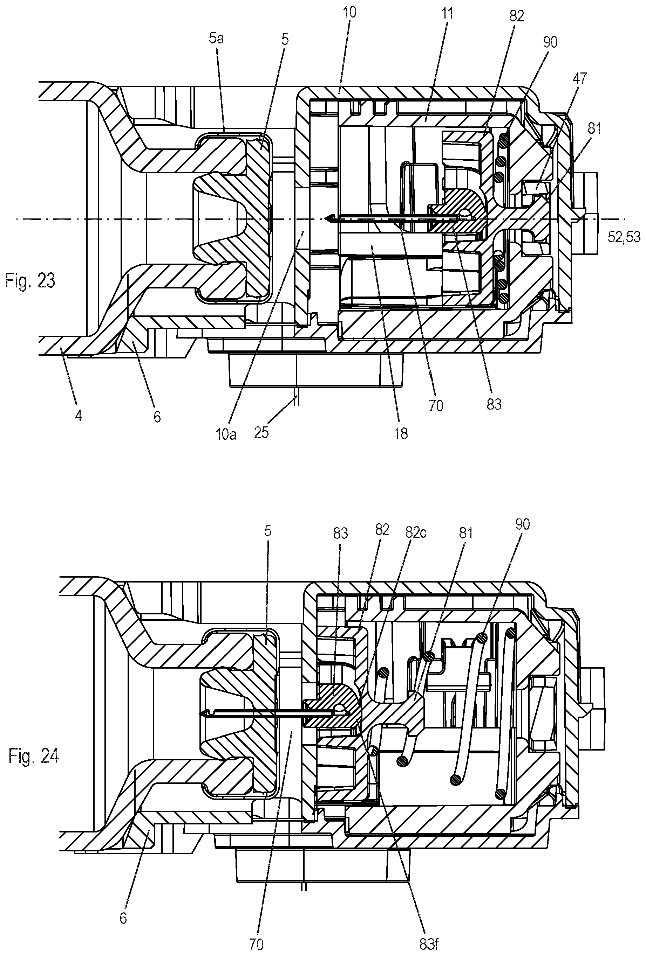

Figure 23: Cross section of cartridge needle insertion mechanism with a complaint coupling mechanism when the cartridge needle holder is in the retracted (first) position,
Figure 24: Cross section of cartridge needle insertion mechanism with a complaint coupling mechanism when the cartridge needle holder is in the inserted (second) position. The needle axis coincides with the insertion axis,

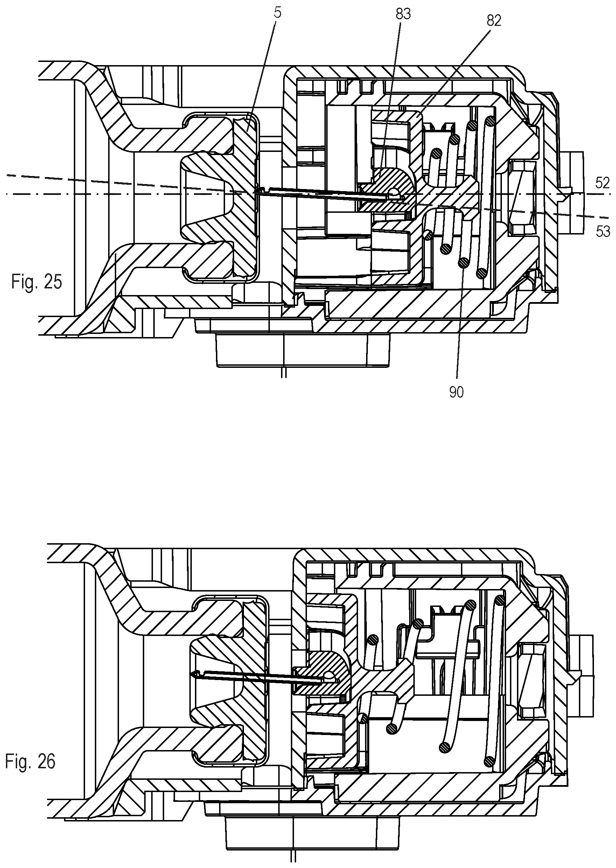

Figure 25: Cross section of cartridge needle insertion mechanism with a complaint coupling mechanism when the cartridge needle holder has left the retracted (first) position. The needle axis is oblique to the insertion axis,
Figure 26: Cross section of cartridge needle insertion mechanism with a complaint coupling mechanism when the cartridge needle holder is in the inserted (second) position. The needle axis is oblique to the insertion axis,

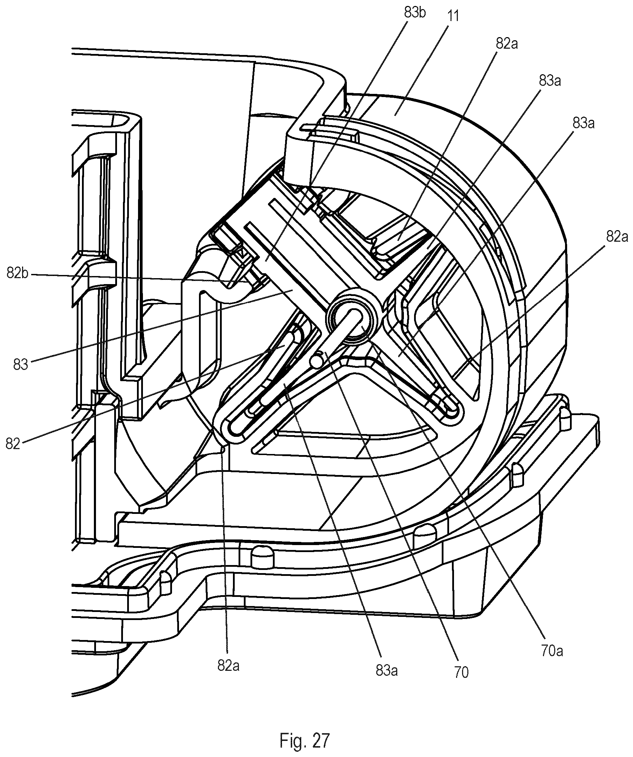

Figure 27: 3D view of the compliant coupling mechanism,

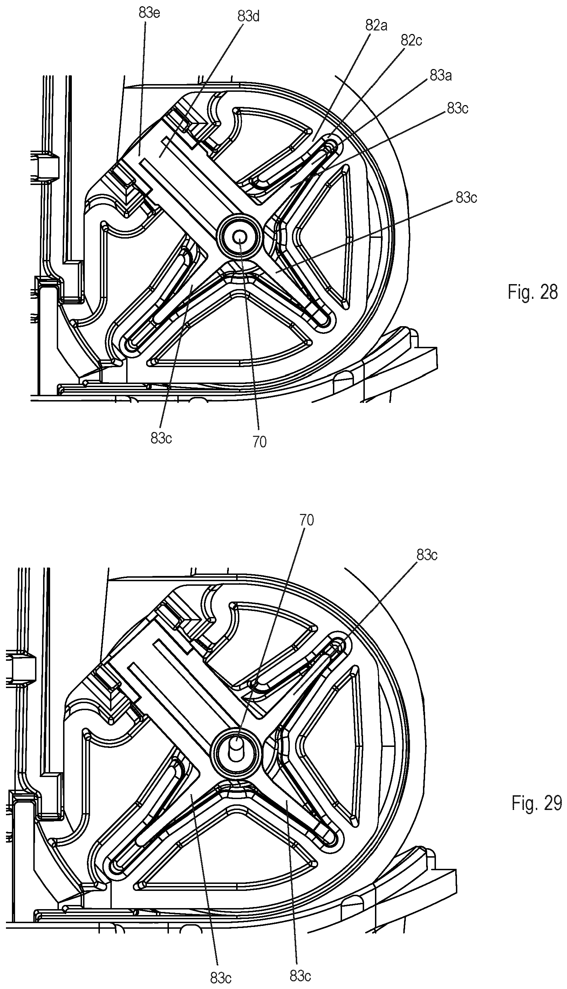

Figure 28: Compliant coupling mechanism non-deformed state, and
Figure 29 Compliant coupling mechanism deformed state.

### DETAILED DESCRIPTION OF PREFERRED EMBODIMENTS

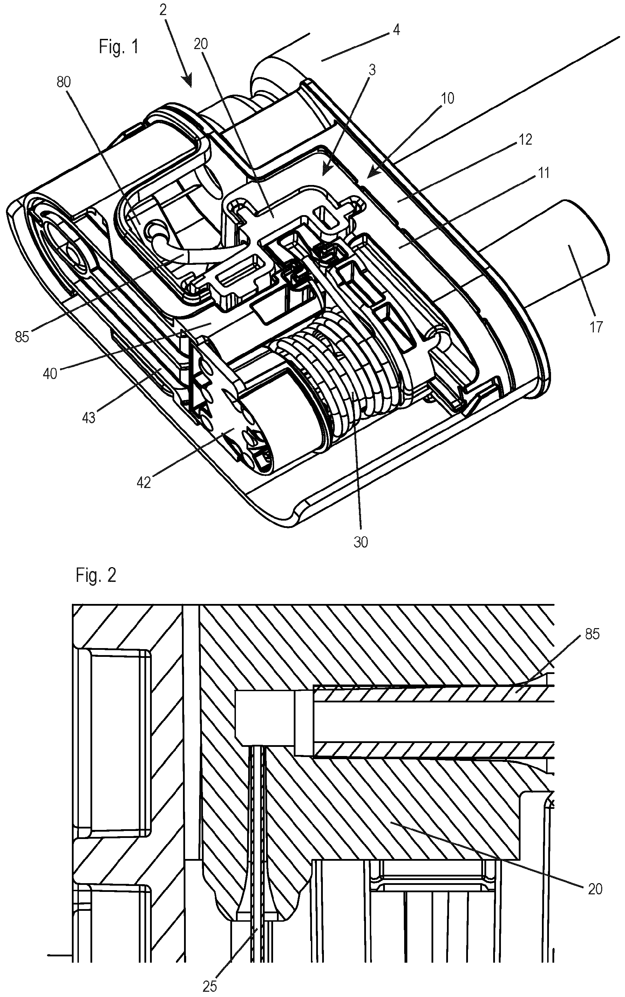

Referring to Figures 1 to 19 an embodiment of a prior art needle insertion and retraction module 2 including a needle insertion and retraction mechanism 3 is disclosed without a compliant coupling mechanism between a cartridge needle holder and cartridge needle slider.

As displayed in Figure 1 the needle insertion and retraction mechanism 3 comprises a housing 10 which is a multiple component housing. Particularly the housing 10 comprises a first housing 11 and a second housing 12 which are connected to each other by positive fit or by firmly bonding or welding. Alternatively the first and the second housings 11, 12 are molded as a single part.

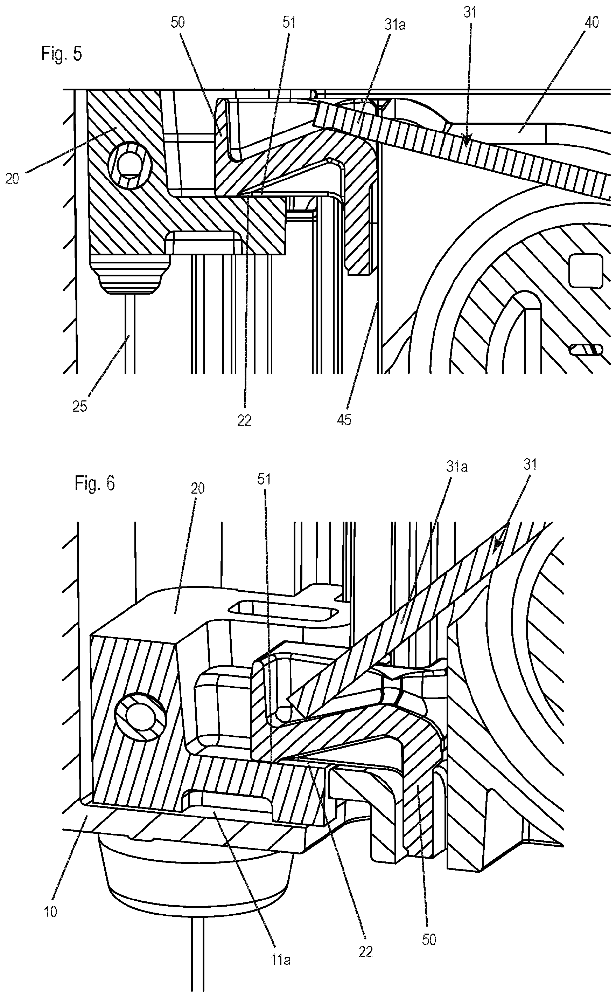

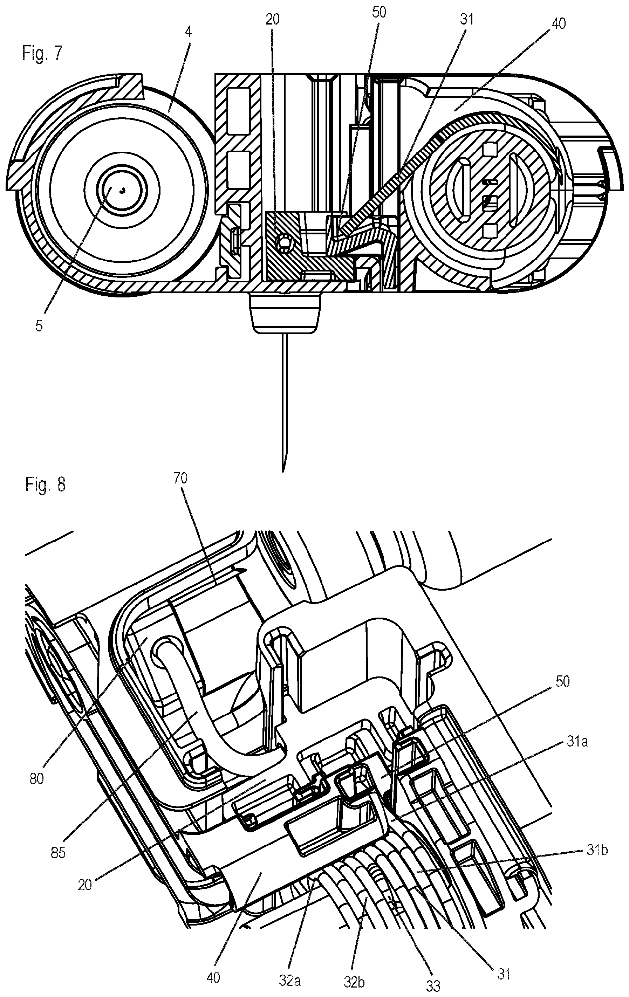

The needle insertion and retraction mechanism 3 comprises a needle carrier 20 which holds a needle 25 (Figure 2) and which is linearly guided by the housing 10, particularly by the first housing 11. The housing 10 or the first housing 11 comprises a longitudinal guide (Figure 1) which engages the needle carrier 20 such that it is movable along the longitudinal axis of the needle 25. The longitudinal guide comprises at least a first longitudinal groove and a second longitudinal groove formed by the housing 11. The needle carrier 20 comprises at least a first rib and a second rib wherein the first rib engages the first groove and the second rib engages the second groove. Thereby the needle carrier 20 is linearly guided to be moved along the longitudinal axis of the needle 25. The needle carrier 20 is movable between an initial position (Figure 1) in which the needle 25 which protrudes from the needle carrier 20 in a needle insertion direction is completely encompassed by the housing, and a needle insertion position (Figures 6 and 7) in which the needle protrudes from an outer surface of the housing, particular the surface which is intended to be contacted or adhered to the skin of a patient. The housing 10 may comprise an opening or a pierceable wall through which the needle 25 is moved when the needle carrier 20 is moved from its initial position to its needle insertion position. The longitudinal axis of the needle 25 is substantially perpendicular or normal with respect to the surface which is intended to be adhered to the skin of the patient. The needle 25 is a hollow needle through which a medication or a medicament can be injected into the patient. The housing 10, particularly the housing 12 is adapted to retain a product container 4. In the example shown, the product container 4 is a cartridge, with a pierceable septum 5 (wall) at its forward end. The medicament of the cartridge 4 can be expelled through a flexible tube 85 which is in fluid communication with the hollow needle 25 and through the needle 25 in a patient. As can be seen in Figure 2 the needle carrier 20 comprises a channel which connects an end of the flexible tube 85 and the hollow needle 25 in a fluid guiding manner. The needle 25 is fixedly retained in a bore of the needle carrier 20. The flexible tube 85 is with one end fixedly retained in a bore of the needle carrier 20.

The other end of the flexible tube 85 is fixedly retained in a bore of a spike carrier 80 (or so-called cartridge needle carrier) which connects a hollow spike 70 (or cartridge needle) in a fluid guiding manner with the flexible tube 85, by means of a channel formed by the spike carrier 80. The spike 70 is fixedly retained in a bore of the spike carrier 80. One end of the flexible tube 85 is fixedly retained in a bore of the spike carrier 80.

The needle insertion and retraction mechanism 3 further comprises a first spring member 31 which is adapted to move the needle carrier 20 with respect to the housing 10 in a needle insertion direction along the longitudinal axis of the needle 25. Furthermore, a second spring member 32 is provided which is adapted to retract the needle carrier 20 with respect to the housing 10 in a needle retraction direction, which is opposed to the needle insertion direction. In the embodiment shown, the first spring member 31 and the second spring member 32 are integrally formed by one spring 30. However, in an alternative, spring members 31 and 32 can be separate from one another.

The first spring member 31 comprises a first helical spring section 31b which operates as a torsion spring (Figure 8). A first arm 31a protrudes from the circumference of the first helical spring section 31b. The first spring member 31 is supported on a control element 40, preferably shaped as a slider, such that the first helical spring section 31b can be strained or tensioned by pivoting the arm 31a. Furthermore, the energy stored in the first helical spring section 31b can be released whereby the first arm 31a is pivoted in a direction which causes the needle carrier 20 to move in the needle insertion direction.

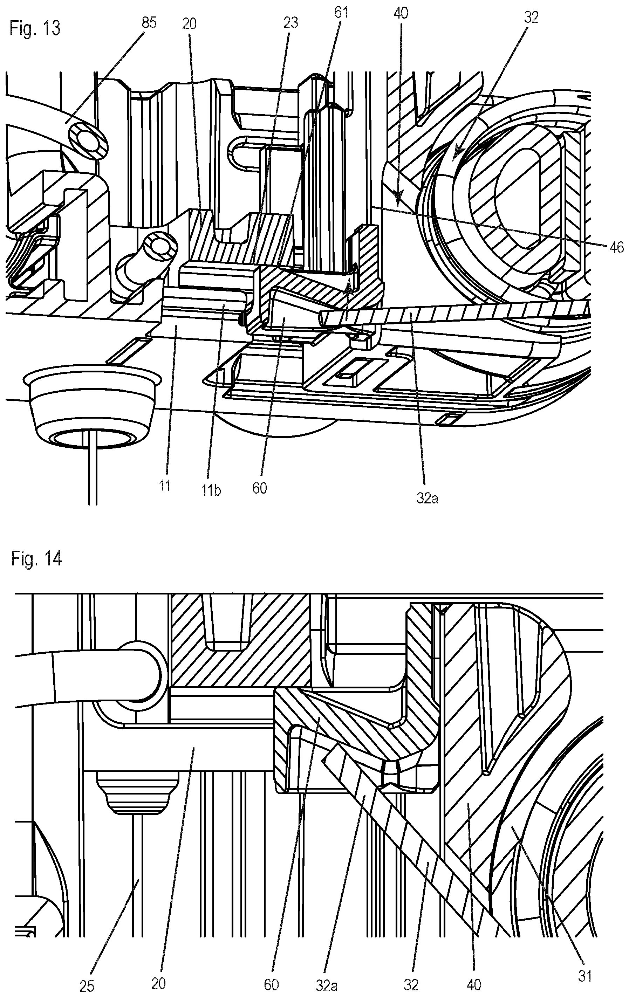

The second spring member 32 (Figure 13) comprises a second helical spring section 32b which operates as a torsion spring. A second arm 32a protrudes from circumference of the second helical spring section 32b. The second spring member 32 is supported on the control element 40 or slider such that the second helical spring section 32b can be strained or tensioned by pivoting the arm 32a. Furthermore, the energy stored in the second helical spring section 32b can be released whereby the second arm 32a is pivoted.

The first helical spring section 31b and the second helical spring section 32b surround a portion of the control element 40. This portion comprises a slit which retains an interconnecting section 33 of the spring 30 which interconnects the first helical spring section 31b and the second helical spring section 32b and which also provides the support section of the first spring member 31 and the second spring member 32 for tensioning the spring sections 31a and 31b. In embodiments with two separate spring members 31 and 32 each of them can comprise a supporting section by which the spring member 31, 32 is supported on the control element 40.

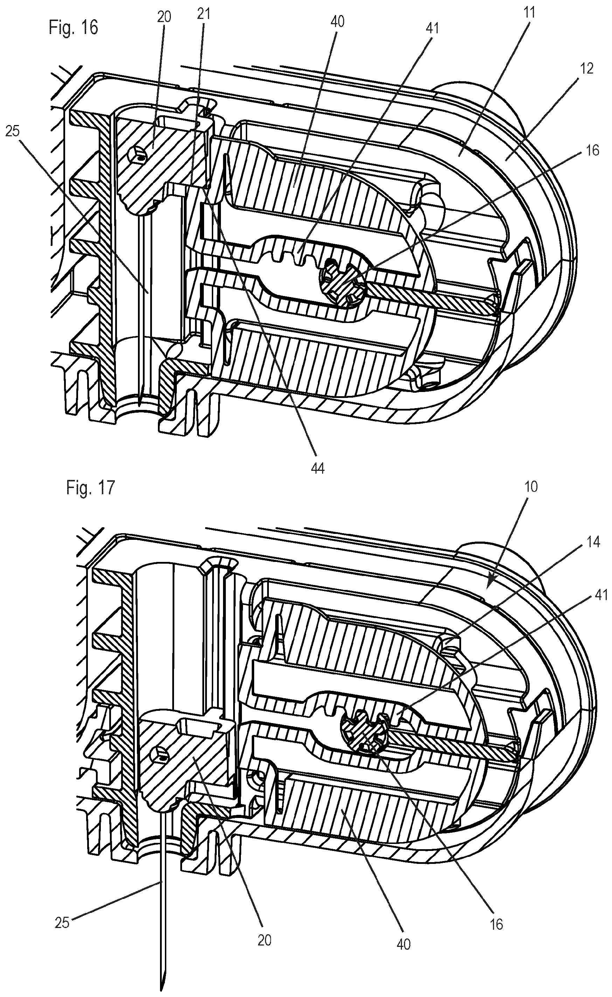

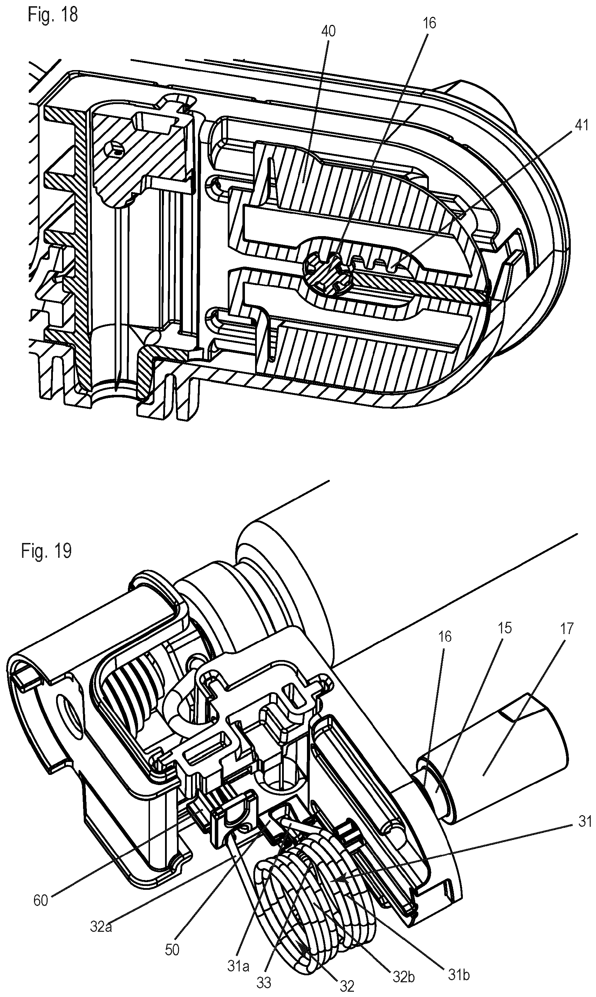

The control element 40, preferably the slider, is linearly guided with respect to the housing 10 to be moved transversely with respect to the longitudinal axis of the needle 25. The control element 40 can be moved from a first position or starting position (Figure 16) to a second position or needle insertion release position (Figure 17). The slider may be moved to a third position, a so-called needle retraction release position (Figure 18). The control element 40 moves from the starting position to the needle retraction release position, including the positions between the starting position and the needle retraction release position, in the same direction. The spring 30 or the spring members 31, 32 are attached to the control element 40 such that they move together with the control element 40. The needle insertion and retraction mechanism 3 or module 2 provides for a drive shaft 15 which is rotatably guided by the housing 10, e.g. by virtue of a rotational bearing (Figure 19). The drive shaft 15 is operatively connected to the control element 40. The drive shaft 15 and the control element 40 are adapted to cooperate with each other such that rotation of the drive shaft 15 in a first rotational direction causes the control element 40 to be linearly moved, namely transversely with respect to the longitudinal axis of the needle 25 because of the linear guidance provided by the housing 10.

The drive shaft 15 comprises a gear wheel 16 (Figures 16 to 18) which is formed by or connected to the drive shaft 15 and which engages a gear rack 41 formed by or connected to the control element 40 to form a rack-and-pinion arrangement. By rotating the drive shaft 15 or the gear wheel 16 the control element 40 is linearly moved.

The drive shaft 15 comprises a coupling member 17 which is adapted to be coupled with a coupling member of a drive shaft of a drive mechanism (not shown). Thereby, rotation of the drive shaft 15 of the drive mechanism in a first direction is transmitted to the drive shaft 15 in the first direction causing the control element 40 to be moved in the first longitudinal direction. The drive shaft 15 is rotated by an active drive, either directly or via a gearing arrangement that may include a worm wheel.

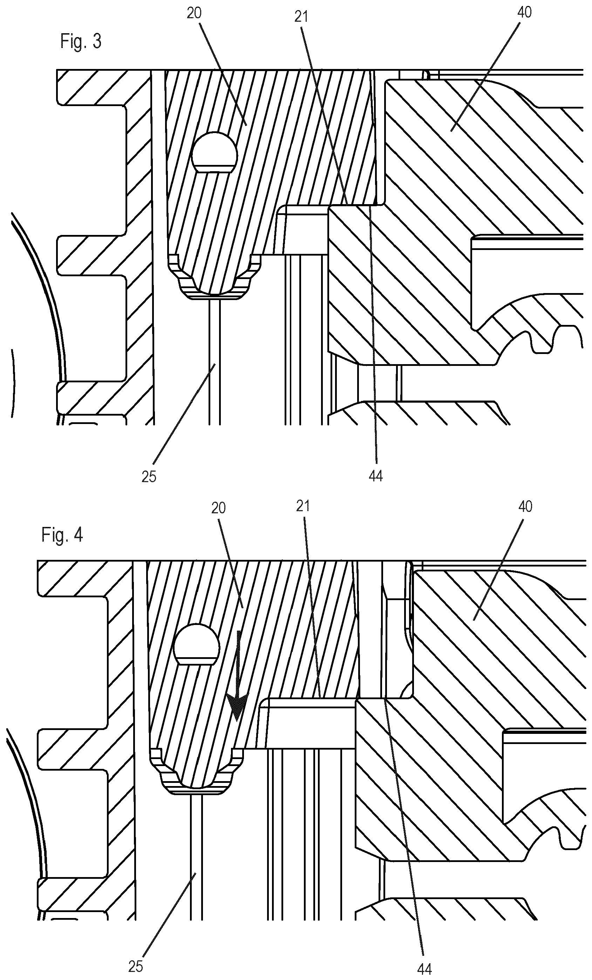

The control element 40 comprises a cap 42 which is connected to a main body 43 of the control element 40 (Figure 1). The cap 42 is connected to or partially fits over the portion which is surrounded by the helical spring sections 31b, 32b. The cap 42 keeps the spring 30 or the spring members 31, 32 in position on the control element 40 or the main body 43 (Figure 1). The control element 40 is operatively coupled to the needle carrier 20 to prevent the needle carrier 20 from being moved in the needle insertion direction when the control element 40 (or slider) is in its starting position (Figure 3). As can be seen in Figure 3, the control element 40 or its main body 43 comprises a stop surface 44 on which a counter stop surface 21 of the needle carrier 20 rests when the control element 40 is in its starting position. The needle carrier 20 is thereby prevented from being moved in the needle insertion direction. As can be seen in Figure 4, the stop surface 44 disengages from the counter stop surface 21 when the control element 40 is moved by the rack-and-pinion arrangement in its insertion release position such that the needle carrier 20 is free to be moved in the needle insertion direction. The first arm 31a or more generally the spring member 31 operates on the needle carrier 20 via a first intermediate member 50 (Figure 5) to drive the needle carrier 20 from the initial position (Figure 5) in the needle insertion direction into a needle insertion position (Figure 6).

A first intermediate member 50 comprises a counter stop surface 51 which engages a stop surface 22 of the needle carrier 20 when the control element 40 is in its starting position and/or in its insertion release position. The first spring member 31 applies a spring force on the first intermediate member 50 which in turn transmits the spring force to the needle carrier 20 as long as the first intermediate member 50 and the needle carrier 20 are in engagement. A spring powered movement of the needle carrier 20 in the needle insertion direction, when the control element 40 is in its starting position, is prevented when stop surface 44 and counter stop surface 21 are engaged (Figure 3). As soon as the control element 40 and the needle carrier 20 are disengaged the first spring member 31 drives the needle carrier 20 in the needle insertion direction until the needle carrier 20 abuts an axial stop 11a provided by the housing 10, particularly by the first housing 11, in the needle insertion position of the needle carrier 20. The control element 40 comprises a linear guide 45 which is adapted to linearly guide the first intermediate member 50 in the direction of the longitudinal axis of the needle 25 or the needle insertion and retraction direction (Figure 5). The linear guide 45 causes the first intermediate member 50 to be moved together with the control element 40 transversely with respect to the longitudinal axis of the needle from the starting position via at least the needle insertion release position to the needle retraction release position. By moving the control element 40 (or slider) from its starting position to its needle insertion release position the first intermediate member 50 is moved with respect to the needle carrier 20 but does not yet disengage from the needle carrier 20. That is to say that the first intermediate member 50 and the needle carrier 20 remain engaged in the needle insertion release position of the control element 40.

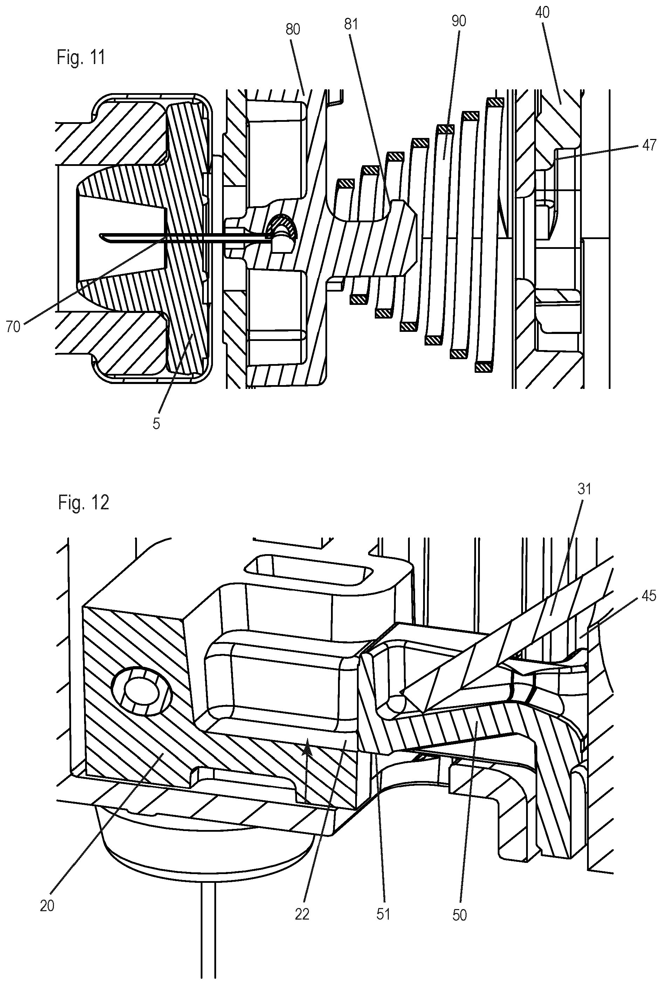

When the control element 40 is moved further from the second slider position by activating the rack and pinion arrangement to its retraction release position (third slider position), the second spring member 32 is operatively coupled to the needle carrier 20 such that the second spring member 32 drives the needle carrier 20 in the needle retraction direction. By moving the control member 40 into the needle retraction release position the first intermediate member 50 and the needle carrier 20, particularly the stop surface 22 and the counter stop surface 51, disengage since the first intermediate member 50 is moved together with the control element 40 transversely with respect to the longitudinal axis of the needle 25. The needle carrier 20 is now free to be moved in the needle retraction direction which is opposed to the needle insertion direction (Figure 12).

For example, when the first intermediate member (50) is disengaged from the needle carrier 20, it - driven by the remainder of the spring force of the first spring member 31 - abuts a stop formed by the control element 40, particularly by the end of the linear guide 45. Thereby, the remainder of the spring force of the first spring member 31 advantageously prevented from interfering with the further operation of the mechanism.

A second intermediate member 60 (Figure 13) is provided, which is linearly guided by the control element 40 in the needle retraction direction, e.g. by a linear guide 46 provided by the control element 40. The linear guide 46 is adapted that the second intermediate member 60 is linearly movable with respect to the control element 40 along the longitudinal axis of the needle 25 or in the needle retraction direction. Furthermore, the linear guide 46 causes the second intermediate member 60 to be moved together with the control element 40 transversely with respect to the needle retraction direction or transversely with respect to the longitudinal axis of the needle 25.

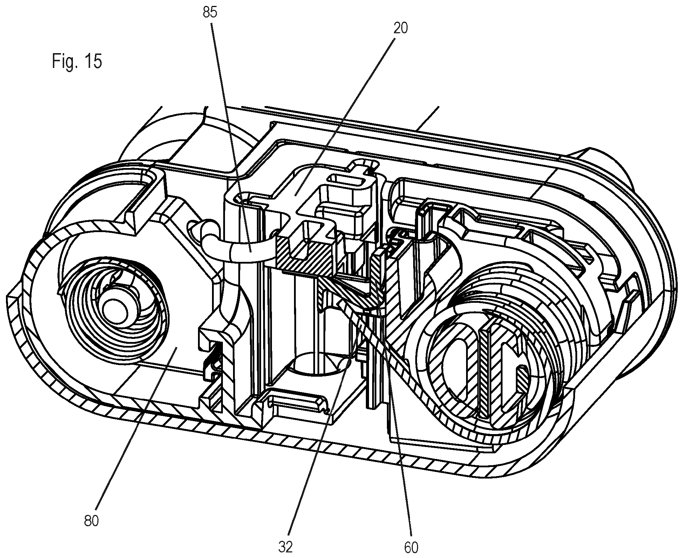

When the control element 40 is in its starting position (first slider position) and/or in its insertion release position (second slider position), the second intermediate member 60 it is engaged with the housing 10, particularly the housing 11, such that the second intermediate member 60 is prevented from being moved in the needle retraction direction (Figure 13). The housing 10 comprises a stop surface 11b with which the second intermediate member 60 is engaged to prevent the second intermediate member 60 from being moved in the needle retraction direction. The second spring member 32 applies a spring force on the second intermediate member 60 in the needle retraction direction. By moving the control element 40 in its retraction release position the second intermediate member 60 is disengaged from the housing 10, particularly from the stop surface 11b. Furthermore, the second intermediate member 60 or a counter stop surface 61 thereof engages with the needle carrier 20 or a stop surface 23 thereof. Thereby, the second intermediate member 60 and the needle carrier 20 are moved in the needle retraction direction driven by the second spring member 32 (Figure 13). Thereby the needle carrier 20 is moved in its retracted position such that the needle 25 is completely retracted into the housing 10 (Figures 14 and 15).

To prevent the first intermediate member 50 and the second intermediate member 60 from interfering with each other they are positioned offset from one another particularly in the direction which is transversal with respect to the longitudinal axis of the needle 25 (Figure 19).

The first spring arm 31a rests on a convexly curved contact surface of the first intermediate member 50 as can be seen in Figure 5. During the first intermediate member 50 is driven by the first spring member 31 in the needle insertion direction, the spring arm 31a, particularly its circumference surface, moves over the apex of the convexly curved contact surface, thereby the first arm 31a (or its circumference surface) particularly slides and/or rolls over over the convexly curved contact surface. This arrangement reduces friction and/or reduces the risk of malfunction with respect to other arrangements.

Movement of the needle carrier 20 in the needle retraction direction is prevented at least by the remainder of the spring force of the first spring member 31 operating on the first intermediate member 50 as long as the first intermediate member 50 is engaged with the needle carrier 20 (Figure 6).

Furthermore, as shown in Figure 6, the free end of the first spring arm 31a comprises an edge, for example, formed between the circumference surface and the end face of the first spring arm 31a. When the needle carrier 20 is in its needle insertion position, the edge contacts or rests on, particularly - to small or microscopically extent - grooves into, the first intermediate member 50, e.g. on an inclined surface thereof. The edge contacting or even grooving into the first intermediate member 50 increases friction between the first intermediate member 50 and the first spring arm 31a. Thereby, movement of the needle carrier 20 in the needle retraction direction is - in addition to the remainder of the spring force of the first spring member 31 - made more difficult or even prevented as long as the first intermediate member 50 is engaged with the needle carrier 20. The angle between the inclined surface and the circumference surface may, for example, be smaller than the angle between the end face and the circumference surface.

The second spring arm 32a rests on a convexly curved contact surface of the second intermediate member 60 as can be seen in Figures 13 and 14. While the second intermediate member 60 is driven by the second spring member 32 in the needle retraction direction, the spring arm 32a moves over the apex of the convexly curved contact surface thereby the second arm 32a particularly slides and/or rolls over over the convexly curved contact surface. This arrangement reduces friction and/or reduces the risk of malfunction with respect to other arrangements.

Movement of the needle carrier 20 back in the needle insertion direction is prevented at least by the remainder of the spring force of the second spring member 32 operating on the second intermediate member 60 as long as the second intermediate member 60 is engaged with the needle carrier 20 (Figure 14).

Furthermore, as shown in Figure 14, the free end of the second spring arm 32a comprises an edge, for example, formed between the circumference surface and the end face of the second spring arm 32a. When the needle carrier 20 is in its needle retraction position, the edge contacts or rests on, particularly - to small or microscopically extent - grooves into, the second intermediate member 60, e.g. on an inclined surface thereof. The edge contacting or even grooving into the second intermediate member 60 increases friction between the second intermediate member 60 and the second spring arm 32a. Thereby, movement of the needle carrier 20 back in the needle insertion direction is - in addition to the remainder of the spring force of the second spring member 32 - made more difficult or even prevented as long as the second intermediate member 60 is engaged with the needle carrier 20. The angle between the inclined surface and the circumference surface may, for example, be smaller than the angle between the end face and the circumference surface.

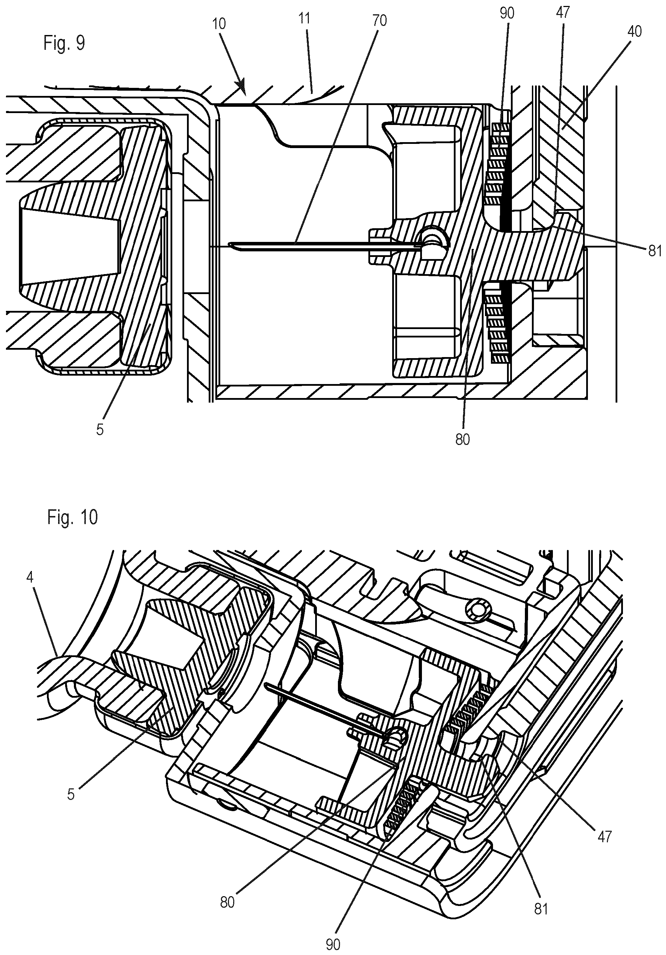

The spike carrier 80 holds a hollow spike 70 which may be also called a cartridge needle 70, which protrudes from the spike carrier 80 towards a receptacle for the product container (cartridge holder) or to a pierceable wall 5 of the cartridge 4 (Figures 8 to 11). In Figures 9 and 10 the spike carrier 80 is in a first position in which the spike 70 does not pierce the wall 5 of the product container 4. The spike carrier 80 is linearly guided, e.g. by a linear guide provided by the housing 10, preferably by the housing 11, such that the spike carrier 80 can be moved linearly from a first position to a second position together with the spike 70. The movement from the first to the second position is along an insertion axis. By moving the spike carrier 80 from the first position to the second position the spike 70 pierces the wall 5 of the product container 4 such that the spike 70 establishes a fluid communication between the medication inside the product container 4 and the needle 25. A spring 90 is provided, which operates on the spike carrier 80 to drive the spike carrier 80 from the first position to the second position. In the first position of the spike carrier 80 the spring 90 is in a pretensioned condition. A variety of spring designs may be conceivable, whereby a conical helical spring 90 is preferred. One end of the spring 90 is supported on the spike carrier 80 and the other end of the spring 90 is supported on the housing 10, particularly the housing 11.

The control element 40 is engaged with the spike carrier 80, when the control element 40 is in its starting position. Thereby, the spike carrier 80 is retained in its first position and the spring 90 is prevented from expanding. Particularly, the control element 40 comprises a retaining surface 47 which engages a counter surface 81 to prevent the spike carrier 80 from being moved from the first position to the second position. By moving the control element 40 (or slider) from its starting position in the first direction, e.g. the direction to the injection release position the control element 40 is disengaged from the spike carrier 80, particularly the retaining surface 47 disengages from the counter surface 81 such that the spike carrier 80 is free to be moved from the first position to the second position (Figure 10). The spring 90 expands and thereby drives the spike carrier 80 from the first position into the second position (Figure 11). The needle insertion and retraction mechanism can be adapted such that the spike carrier 80 is released before, after or at the same time the needle carrier 20 is released to be moved in the needle insertion direction.

The spike carrier 80 comprises a main body which holds the spike 70 (or cartridge needle 70) and which is linearly guided by the housing 10. The cartridge needle may be a hollow steel needle or made from a suitable plastic material. A hollow steel needle may be glued into the spike carrier 80. The spike carrier 80 comprises a protrusion which protrudes from the main body opposite to the direction in which the spike 70 protrudes. The protrusion extends through the (conical) helical spring 90, through a section of the housing 10. The section of the housing 10 can be arranged between the section of the control element 40 which comprises the retaining surface 47, and the spike carrier 80. The protrusion comprises the counter surface 81.

Figures 20 a-d shows cartridge a needle insertion with the cartridge needle axis 53 parallel to the insertion axis 52 using a cartridge needle insertion mechanism according to the embodiments disclosed in Figures 1 to 19. Figures 21a-d shows cartridge needle insertion with the cartridge needle axis 53 oblique to the insertion axis 52 with the cartridge needle insertion mechanism according to the embodiments disclosed in Figures 1 to 19.

Figures 22 a-d shows cartridge needle insertion with the cartridge needle axis 53 oblique to the insertion axis 52 with a cartridge needle insertion mechanism having a compliant coupling between a cartridge needle holder and a cartridge needle slider according to the embodiments that will be presented in Figures 23 to 29. The force-displacement curves for the forces along (N), and forces perpendicular (Q) to the insertion axis 52 are presented in the schematic diagrams next to each Figures a-c.

When a cartridge needle 70 is inserted with its needle axis 53 being equal to the insertion axis 52, then there are no perpendicular forces Q acting on the needle 70, Figures 20a-20c, and the penetration hole in the septum approximately equals the cross section of the cartridge needle, see Figure 20d. The forces N acting along the insertion axis initially rise (Figure 20a) as the tip of the needle penetrates the surface of the septum 5, and the force N reaches a maximum value once the tip fully penetrates through the septum (Figure20b).The frictional forces between the outside surface of the needle 70 and the inside surface of the elastically deformed penetration hole ensures that a constant normal force N needs to be supplied by the insertion mechanism (Figure 20c).

When a cartridge needle 70 is inserted with its needle axis 53 being angulated to the insertion axis 52, then perpendicular forces Q will act on the needle 70, Figures 21a-21c, and the penetration hole in the septum is larger compared to the ideal situation described above where the needle axis is perpendicular to the septum (compare Figures 20d and 21d). The cross sectional area of the passage in the septum 5 is enlarged as the angulated (and sharp) needle tip cuts through the septum (Figure 21d). The force profile for the penetration forces N along the insertion axis is more or less equal to the profile for a parallel insertion, compare the force profiles N in Figures 20c and 21c. For the off-axis insertion, needle penetration forces perpendicular the insertion axis will occur and those are schematically represented by a non-linear curve rising to a maximum value once the needle has fully penetrated through the septum (Figure 21c).

In the situation where the needle penetration forces Q oriented perpendicular to the insertion axis are compensated for, then the off-axis penetration forces are reduced (Figure 22c). The needle penetration forces Q are reduced but not completely eliminated as the off-axis insertion angle is not eliminated since the insertion mechanism is not an alignment mechanism. The flexibility in the insertion mechanism comprising the compliant coupling ensures that cartridge needle tip is not pushed through the septum (thereby cutting a larger hole as in Figure 21d), but, nicely cuts through the septum as the center of the cartridge needle holder is moved with respect to the cartridge needle slider thereby minimizing the hole in the septum and thereby minimizing the leakage risk.

An embodiment for a needle insertion mechanism with a compliant coupling will be presented in the following Figures 23 to Figure 29. The cartridge needle carrier or spike carrier 80 in the previous embodiment (Figure 10) has been split into two separate parts, a cartridge needle holder 83 and a cartridge needle slider 82. The cartridge needle holder 83 holds the proximal section of the needle 70 opposite from the needle tip. The cartridge needle slider 82, is engaged with the housing 10,11 using a linear guidance. The linear guidance may be oriented parallel to the insertion axis 52, and preferably parallel to the longitudinal axis of the cartridge. The housing comprises a linear groove 18 engaging a not shown linear protrusion on the cartridge needle slider 82. The groove-protrusion combination may be reversed as well and there may be a plurality of linear guidances guiding the cartridge needle slider 82 along the insertion axis 52. The cartridge needle holder 83 comprises a bore for receiving the proximal section of the needle and the needle may be fixed inside the bore using a press-fit connection, a glue, barbed hooks extending from the needle or the plastic material for the cartridge needle holder is injection moulded around the cartridge needle 70. In case a glue is used this may be a flexible glue (after curing) providing an extra dampening effect during insertion through the septum 5 into the cartridge. The bore in the cartridge needle holder is directly or indirectly connected to the tubing 85 of the fluid conduit. The section of the cartridge needle holder 83 that is opposite to the bore is closed and has a stop surface 83f abutting a counter stop surface 82c on the cartridge needle slider 82. The spring 90 is compressed between the housing 11 and the cartridge needle slider 82 as the counter surface 81 of the cartridge needle slider engages the retaining surface 47 of the slider 40 to keep the cartridge needle 70 in the retracted position (Figure 23). When the slider 40 is moved by rotation of the cam shaft 15 activating the rack- and-pinion 16,41 (Figures 16 and 17), then the engagement 47,81 between the slider 40 and the cartridge needle slider 83 is released and spring 90 decompressed. The spring 90 pushes on the cartridge needle slider 83 and due to the abutment 82c, 83f, the cartridge needle holder 83 will be forced to moved together with the cartridge needle slider 82 towards the cartridge 6 while the cartridge needle slider 82 is guided by the linear guidance 18. The abutment 82c, 83f ensures that needle penetration forces N, along the insertion axis are absorbed as the needle penetrates the septum. The tip of the cartridge needle 70 passes through passage 10a and penetrates through the septum 5 that is held onto the cartridge 4 by a crimp 5a. In Figures 23 and 24, the axis for the needle 53 is parallel to the insertion axis 52 whereas an off-axis insertion is shown in Figures 25 and 26 where the needle axis 53 has an inclination angle with respect to the insertion axis 52. Details for the compliant coupling between the cartridge needle slider 82 and the cartridge needle holder 83 are shown in Figures 27 to 29.

The cartridge needle holder 83 comprises four first coupling members 83a, 83b shaped as protrusions that radially extend outwards from the cartridge needle axis. Three first coupling members 83a comprise a flexible arm 83c whereas one of the four first coupling members 83b is rigid and comprises a passage 83e for connection to the tubing 85 of the fluid path. The passage 83e connects the proximal end of the cartridge needle 70 to the (not shown) tubing. The four first coupling members 83a, 83b each engage one of four second coupling members 82a, 82b on the cartridge needle slider 82. Three of the four second coupling members 82a are configured to receive the first coupling members 83a comprising the flexible arm 83c whereas one of the four second coupling members 82b engages the rigid first coupling member 83b comprising the passage 83e. The three second coupling members 82a are shaped as a longitudinal recess or pocket. All four second coupling members are designed to be rigid or semi-rigid when engaging the four first coupling members. The engagement between the first coupling members 83a comprising the flexible arm 83c and the rigid walls in the recess or pocket of the second coupling members 82a is such that there may be a frictional engagement when the coupling members move or slide with respect to each other. Optionally, the flexible arms 83c may bend when engaging the second coupling members 82a. The frictional and/or elastic forces required are used to compensate for needle penetration forces as the cartridge needle slider 82 moves from the first (not inserted) to the second (inserted) position. The first coupling member 83b comprising the passage 83e is designed such that there may be relative rotation or tilting with respect to the rigid second coupling member 82b without any elastic deformation. The second coupling member 82b may be shaped as a cut out in the cartridge needle slider. The first coupling members 83a are preferably shaped as wings or blades that radially extend outwards and engage complementary pockets of the second coupling members 82a. The wings or blades of the first coupling members 83a are preferably oriented parallel to the insertion axis, optionally, one or all of the three first coupling members 83a may be tilted with respect to the insertion axis. The surfaces of the first and second coupling surfaces 83a, 82a may be textured, roughened or sand blasted to fine tune the frictional engagement. Alternatively a lubricant, for example a silicone oil, may be used to reduce friction. The first and second coupling members 83a, 82a are preferably made from a polymeric material that may be friction optimized by adding a friction agent such as silicone oil, teflon, fluoro(co)polymers or an olefinic wax. The cartridge needle holder 83 may be snap-fitted into the cartridge needle slider 82 using hooks, catches or flexible arms on the cartridge needle slider and/or the cartridge needle holder. The snap-fit connection improves handling during assembly and reduces the play between the stop surface 83f and the counter stop surface 82c. In Figure 28, the compliant coupling arrangement between the cartridge needle holder 83 and the cartridge needle slider 82 in the non-deformed state where the cartridge needle 70 inserts parallel to the insertion axis. Optionally, the cartridge needle 70 is adhesively connected to the cartridge needle holder 83 using a flexible glue 70a. For an off-axis insertion, the compliant coupling is shown in the deformed state in Figure 29.

Variations of the embodiments presented in Figures 23 to 29 can be envisaged without deviating from the concept of the invention. For example, the number of first and second coupling members forming pairs of coupling members may be carried, for example two, three or five pairs can be envisaged. Also the number of rigid and flexible coupling members may be varied. It may be that the cartridge needle slider comprises two separate cartridge needles and the cartridge needle may be a pencil tip needle.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims.

**LIST OF REFERENCE SIGNS**

| | | | |
|---|---|---|---|
| 1 | drive module | 23 | stop surface |
| 2 | insertion and retraction module | 25 | needle, skin insertion needle |
| 3 | insertion and retraction mechanism | 30 | spring/lever spring |
| 4 | product container, cartridge | 31 | first spring member |
| 5 | pierceable wall/septum | 31a | first spring arm |
| 5a | crimp | 31b | first helical spring section |
| 6 | housing, cartridge holder | 32 | second spring member |
| 10 | housing | 32a | second spring arm |
| 10a | passage | 32b | second helical spring section |
| 11 | first housing | 33 | interconnecting section |
| 11a | axial stop | 40 | control element / slider |
| 11b | stop surface | 41 | gear rack / rack |
| 12 | second housing | 42 | cap |
| 13 | linear guide | 43 | main body |
| 14 | linear guide | 44 | (first) stop surface |
| 15 | drive shaft, cam-shaft | 45 | linear guide |
| 16 | gear wheel / pinion | 46 | linear guide |
| 17 | coupling member | 47 | retaining surface |
| 18 | linear guide /groove | 50 | first intermediate member |
| 20 | needle carrier, needle holder | 51 | counter stop surface |
| 21 | counter stop surface | 52 | Insertion axis |
| 22 | stop surface | 53 | Cartridge needle axis |
| 60 | second intermediate member | 83 | cartridge needle holder |
| 61 | counter stop surface | 83a | first coupling member |
| 70 | hollow spike, cartridge needle | 83b | first coupling member |
| 70a | flexible connector | 83c | flexible arm |
| 80 | spike carrier, cartridge needle carrier | 83d | rigid arm |
| 81 | counter surface | 83e | passage |
| 82 | cartridge needle slider | 83f | stop surface |
| 82a | second coupling member | 85 | flexible tube |
| 82b | second coupling member | 90 | spring |
| 82c | counter stop surface | | |

## Claims

1. An assembly for a cartridge needle insertion mechanism comprising:
a housing (10, 11),
a cartridge needle (70) defining a cartridge needle axis (53),
a cartridge (4) closed by a septum (5),
a driver (90),
a cartridge needle slider (82), linearly guided by the housing (10,11) along an insertion axis (52) to be moved by the driver from a first position where the cartridge needle (70) is not penetrating the septum (5) to a second position where the cartridge needle (70) penetrates the septum (5) of the cartridge (4),
a cartridge needle holder (83) holding the cartridge needle (70) and being operatively coupled with the cartridge needle slider (82) to be moved together with the cartridge needle slider along the insertion axis (52) from the first to the second position,
**characterized by** a compliant coupling between the cartridge needle holder (83) and the cartridge needle slider (82) configured to facilitate movement of the cartridge needle holder (83) with respect to the cartridge needle slider (82) when moved into the second position, thereby compensating for needle penetration forces that are oriented perpendicular to the insertion axis (52), wherein the compliant coupling comprises a first coupling member (83a, 83b) on the cartridge needle holder (83) engaging a second coupling member (82a, 82b) on the cartridge needle slider (82) in a plane parallel to the insertion axis (52), and wherein the first coupling member (83a, 83b) and the second coupling member (82a, 82b) elastically engage each other and/or are in a linear or rotational friction fit engagement.

2. The assembly according to claim 1 wherein the movement of the cartridge needle holder (83) with respect to the cartridge needle slider (82) is perpendicular to the insertion axis (52), or the movement is a pivoting movement of the cartridge needle holder (83) around the insertion axis (52) or the cartridge needle holder (83) rotates around the insertion axis (52).

3. The assembly according to claims 1 or 2 wherein the first coupling member (83a, 83b) comprises a protrusion radially extending from the cartridge needle holder (83) whereby the protrusion is at least partially elastically and/or plastically deformable and engages the second coupling member (82a, 82b) shaped as a rigid surface on the cartridge needle slider (82) being oriented parallel to the insertion axis (52), or the first coupling member (83a, 83b) comprises a rigid protrusion radially extending from the cartridge needle holder (83) engaging the second coupling member (82a, 82b) shaped as an at least partially elastically and/or plastically deformable surface on the cartridge needle slider (82) being oriented parallel to the insertion axis (52).

4. The assembly according to any of the previous claims wherein the cartridge (4) comprises a barrel defining a cartridge axis and the cartridge (4) is in a fixed position with respect to the housing (10,11) and wherein the cartridge axis is aligned with the insertion axis (52) when the cartridge needle slider (82) is in the first position.

5. The assembly according to claim 4 wherein the assembly is not an alignment arrangement configured to align the cartridge axis and the insertion axis (52) when the cartridge needle slider (82) moves from the first position to the second position.

6. The assembly according to any of the previous claims wherein the cartridge needle slider (82) and the cartridge needle holder (83) are coaxially arranged around the insertion axis (52) when the cartridge needle slider is in the first position.

7. The assembly according to claim 6 wherein the cartridge needle holder (83) is moved out of the coaxial arrangement with respect to the cartridge needle slider (82) when the needle axis (53) is not parallel to the insertion axis (52) when the cartridge needle slider (82) is moved into the second position thereby generating needle penetration forces perpendicular to the insertion axis (52) deforming the compliant coupling.

8. The assembly according to any of the previous claims wherein the cartridge needle holder (83) is axially fixed to the cartridge needle slider (82) while allowing movement with respect to the cartridge needle slider (82) perpendicular to the insertion axis (52), or a pivoting or a rotational movement around the insertion axis (53) against a bias or resistance of the compliant coupling and wherein the cartridge needle slider (82) is linearly guided by the housing (10, 11) by a splined engagement defining the insertion axis.

9. The assembly according to any of the previous claims wherein the cartridge needle (70) is connected to the cartridge needle holder (83) by an elastic member (70a) preferably being an elastic glue.

10. The assembly according to any of the previous claims wherein the driver is a spring (90) located between the housing (10,11) and the cartridge needle slider (82) and acting on the housing (10,11) and the cartridge needle slider (83), the spring (90) being retained in a compressed state to provide a biasing force on the housing (10,11) and the cartridge needle slider (82) when the cartridge needle slider (82) is held in the first position with respect to the housing by a releasable locking feature.

11. The assembly according to any of the previous claims wherein the first coupling member (83a, 83b) is hollow and forms a passage for a fluid conduit establishing a fluid link between a medicament present in the cartridge (4) and the cartridge needle (70).

12. The assembly according to any of the previous claims wherein the cartridge needle (70) is a hollow needle whereby the tip of the needle is sharpened having a closed end such as a pencil tip needle or the tip of the needle is sharpened and having an open end.

13. An injection device comprising the assembly according to any of the previous claims wherein the injection device is a prefilled patch injection device comprising a skin adhesive surface and a skin needle (25) configured to move from a needle retracted position in the housing (10,11) to a needle inserted position at least partially outside of the housing after the cartridge needle slider (82) has moved from the first position to the second position.

## Patentansprüche

1. Anordnung für einen Kartuschennadeleinführmechanismus, umfassend:
ein Gehäuse (10, 11),
eine Kartuschennadel (70), die eine Kartuschennadelachse (53) definiert,
eine Kartusche (4), die durch ein Septum (5) verschlossen ist,
einen Treiber (90),
einen Kartuschennadelschieber (82), der durch das Gehäuse (10,11) entlang einer Einführachse (52) linear geführt wird, um durch den Treiber von einer ersten Position, in der die Kartuschennadel (70) das Septum (5) nicht durchdringt, in eine zweite Position bewegt zu werden, in der die Kartuschennadel (70) das Septum (5) der Kartusche (4) durchdringt,
einen Kartuschennadelhalter (83), der die Kartuschennadel (70) hält und mit dem Kartuschennadelschieber (82) wirkgekoppelt ist, um zusammen mit dem Kartuschennadelschieber entlang der Einführachse (52) von der ersten in die zweite Position bewegt zu werden,
**gekennzeichnet durch** eine nachgiebige Kopplung zwischen dem Kartuschennadelhalter (83) und dem Kartuschennadelschieber (82), die konfiguriert ist, um die Bewegung des Kartuschennadelhalters (83) in Bezug auf den Kartuschennadelschieber (82) zu erleichtern, wenn in die zweite Position bewegt, wodurch Nadeldurchdringungskräfte kompensiert werden, die senkrecht zu der Einführachse (52) ausgerichtet sind, wobei die nachgiebige Kopplung ein erstes Kopplungselement (83a, 83b) an dem Kartuschennadelhalter (83) umfasst, das mit einem zweiten Kopplungselement (82a, 82b) an dem Kartuschennadelschieber (82) in einer Ebene parallel zu der Einführachse (52) in Eingriff steht, und wobei das erste Kopplungselement (83a, 83b) und das zweite Kopplungselement (82a, 82b) elastisch ineinander greifen und/oder in einem linearen oder rotatorischen Reibschlusseingriff stehen.

2. Anordnung nach Anspruch 1, wobei die Bewegung des Kartuschennadelhalters (83) in Bezug auf den Kartuschennadelschieber (82) senkrecht zu der Einführachse (52) ist oder die Bewegung eine Schwenkbewegung des Kartuschennadelhalters (83) um die Einführachse (52) herum ist oder der Kartuschennadelhalter (83) um die Einführachse (52) herum rotiert.

3. Anordnung nach Anspruch 1 oder 2, wobei das erste Kopplungselement (83a, 83b) einen Vorsprung umfasst, der sich radial von dem Kartuschennadelhalter (83) erstreckt, wobei der Vorsprung mindestens teilweise elastisch und/oder plastisch verformbar ist und mit dem zweiten Kopplungselement (82a, 82b) in Eingriff steht, das als eine starre Oberfläche auf dem Kartuschennadelschieber (82) geformt ist, der parallel zu der Einführachse (52) ausgerichtet ist, oder das erste Kopplungselement (83a, 83b) einen starren Vorsprung umfasst, der sich von dem Kartuschennadelhalter (83) radial erstreckt, der mit dem zweiten Kopplungselement (82a, 82b) in Eingriff steht, das als eine mindestens teilweise elastisch und/oder plastisch verformbare Oberfläche an dem Kartuschennadelschieber (82) geformt ist, der parallel zu der Einführachse (52) ausgerichtet ist.

4. Anordnung nach einem der vorstehenden Ansprüche, wobei die Kartusche (4) einen Zylinder umfasst, der eine Kartuschenachse definiert, und sich die Kartusche (4) in einer festen Position in Bezug auf das Gehäuse (10,11) befindet, und wobei die Kartuschenachse mit der Einführachse (52) abgestimmt ist, wenn sich der Kartuschennadelschieber (82) in der ersten Position befindet.

5. Anordnung nach Anspruch 4, wobei die Anordnung keine Abstimmungsanordnung ist, die konfiguriert ist, um die Kartuschenachse und die Einführachse (52) abzustimmen, wenn sich der Kartuschennadelschieber (82) von der ersten Position in die zweite Position bewegt.

6. Anordnung nach einem der vorstehenden Ansprüche, wobei der Kartuschennadelschieber (82) und der Kartuschennadelhalter (83) um die Einführachse (52) herum koaxial angeordnet sind, wenn sich der Kartuschennadelschieber in der ersten Position befindet.

7. Anordnung nach Anspruch 6, wobei der Kartuschennadelhalter (83) aus der koaxialen Anordnung in Bezug auf den Kartuschennadelschieber (82) bewegt wird, wenn die Nadelachse (53) nicht parallel zu der Einführachse (52) ist, wenn der Kartuschennadelschieber (82) in die zweite Position bewegt wird, wodurch Nadeldurchdringungskräfte senkrecht zu der Einführachse (52) erzeugt werden, die die nachgiebige Kopplung verformen.

8. Anordnung nach einem der vorstehenden Ansprüche, wobei der Kartuschennadelhalter (83) axial an dem Kartuschennadelschieber (82) befestigt ist, während eine Bewegung in Bezug auf den Kartuschennadelschieber (82) senkrecht zu der Einführachse (52) oder eine Schwenk- oder eine Rotationsbewegung um die Einführachse (53) herum gegen eine Vorspannung oder einen Widerstand der nachgiebigen Kopplung zugelassen wird, und wobei der Kartuschennadelschieber (82) durch einen Keilwelleneingriff, der die Einführachse definiert, linear durch das Gehäuse (10, 11) geführt wird.

9. Anordnung nach einem der vorstehenden Ansprüche, wobei die Kartuschennadel (70) mit dem Kartuschennadelhalter (83) durch ein elastisches Element (70a) verbunden ist, das vorzugsweise ein elastischer Klebstoff ist.

10. Anordnung nach einem der vorstehenden Ansprüche, wobei der Treiber eine Feder (90) ist, die sich zwischen dem Gehäuse (10,11) und dem Kartuschennadelschieber (82) befindet und auf das Gehäuse (10,11) und den Kartuschennadelschieber (83) wirkt, wobei die Feder (90) in einem komprimierten Zustand gehalten wird, um eine Vorspannkraft auf das Gehäuse (10,11) und den Kartuschennadelschieber (82) auszuüben, wenn der Kartuschennadelschieber (82) durch ein lösbares Verriegelungsmerkmal in der ersten Position in Bezug auf das Gehäuse gehalten wird.

11. Anordnung nach einem der vorstehenden Ansprüche, wobei das erste Kopplungselement (83a, 83b) hohl ist und einen Durchgang für eine Fluidleitung ausbildet, die eine Fluidverknüpfung zwischen einem Medikament, das in der Kartusche (4) vorhanden ist, und der Kartuschennadel (70) herstellt.

12. Anordnung nach einem der vorstehenden Ansprüche, wobei die Kartuschennadel (70) eine Hohlnadel ist, deren Spitze geschärft ist, die ein geschlossenes Ende aufweist, wie eine Bleistiftspitzennadel, oder die Spitze der Nadel geschärft ist und ein offenes Ende aufweist.

13. Injektionsvorrichtung, umfassend die Anordnung nach einem der vorstehenden Ansprüche, wobei die Injektionsvorrichtung eine vorgefüllte Patch-Injektionsvorrichtung ist, umfassend eine Hautklebeoberfläche und eine Hautnadel (25), die konfiguriert ist, um sich aus einer Nadeleinzugsposition in dem Gehäuse (10,11) in eine Nadeleinführposition mindestens teilweise außerhalb des Gehäuses zu bewegen, nachdem sich der Kartuschennadelschieber (82) aus der ersten Position in die zweite Position bewegt hat.

## Revendications

1. Ensemble pour un mécanisme d'insertion d'aiguille de cartouche comprenant :
un boîtier (10, 11),
une aiguille de cartouche (70) définissant un axe d'aiguille de cartouche (53),
une cartouche (4) fermée par un septum (5),
un dispositif d'entraînement (90),
un curseur d'aiguille de cartouche (82), guidé linéairement par le boîtier (10, 11) le long d'un axe d'insertion (52) pour être déplacé par le dispositif d'entraînement d'une première position où l'aiguille de cartouche (70) ne pénètre pas dans le septum (5) à une seconde position où l'aiguille de cartouche (70) pénètre dans le septum (5) de la cartouche (4),
un support d'aiguille de cartouche (83) maintenant l'aiguille de cartouche (70) et accouplé de manière opérationnelle au curseur d'aiguille de cartouche (82) pour être déplacé conjointement avec le curseur d'aiguille de cartouche le long de l'axe d'insertion (52) de la première à la seconde position,
**caractérisé par** un accouplement souple entre le support d'aiguille de cartouche (83) et le curseur d'aiguille de cartouche (82) conçu pour faciliter le mouvement du support d'aiguille de cartouche (83) par rapport au curseur d'aiguille de cartouche (82) lorsqu'il est déplacé dans la seconde position, permettant ainsi de compenser des forces de pénétration d'aiguille qui sont orientées perpendiculairement à l'axe d'insertion (52), dans lequel l'accouplement souple comprend un premier élément d'accouplement (83a, 83b) sur le support d'aiguille de cartouche (83) venant en prise avec un second élément d'accouplement (82a, 82b) sur le curseur d'aiguille de cartouche (82) dans un plan parallèle à l'axe d'insertion (52), et dans lequel le premier élément d'accouplement (83a, 83b) et le second élément d'accouplement (82a, 82b) viennent en prise élastiquement l'un avec l'autre et/ou sont en prise par un ajustement serré linéaire ou rotatif.

2. Ensemble selon la revendication 1, dans lequel le mouvement du support d'aiguille de cartouche (83) par rapport au curseur d'aiguille de cartouche (82) est perpendiculaire à l'axe d'insertion (52), ou le mouvement est un mouvement de pivotement du support d'aiguille de cartouche (83) autour de l'axe d'insertion (52) ou le support d'aiguille de cartouche (83) tourne autour de l'axe d'insertion (52).

3. Ensemble selon les revendications 1 ou 2, dans lequel le premier élément d'accouplement (83a, 83b) comprend une saillie s'étendant radialement à partir du support d'aiguille de cartouche (83), moyennant quoi la saillie est au moins partiellement déformable élastiquement et/ou plastiquement et venant en prise avec le second élément d'accouplement (82a, 82b) sous la forme d'une surface rigide sur le curseur d'aiguille de cartouche (82) orienté parallèlement à l'axe d'insertion (52), ou le premier élément d'accouplement (83a, 83b) comprend une saillie rigide s'étendant radialement à partir du support d'aiguille de cartouche (83) et venant en prise avec le second élément d'accouplement (82a, 82b) sous la forme d'une surface au moins partiellement déformable élastique et/ou plastiquement sur le curseur d'aiguille de cartouche (82) orienté parallèlement à l'axe d'insertion (52).

4. Ensemble selon l'une quelconque des revendications précédentes, dans lequel la cartouche (4) comprend un cylindre définissant un axe de cartouche et la cartouche (4) est dans une position fixe par rapport au boîtier (10, 11) et dans lequel l'axe de cartouche est aligné avec l'axe d'insertion (52) lorsque le curseur d'aiguille de cartouche (82) est dans la première position.

5. Ensemble selon la revendication 4, dans lequel l'ensemble n'est pas un agencement d'alignement configuré pour aligner l'axe de cartouche et l'axe d'insertion (52) lorsque le curseur d'aiguille de cartouche (82) se déplace de la première position à la seconde position.

6. Ensemble selon l'une quelconque des revendications précédentes, dans lequel le curseur d'aiguille de cartouche (82) et le support d'aiguille de cartouche (83) sont agencés coaxialement autour de l'axe d'insertion (52) lorsque le curseur d'aiguille de cartouche est dans la première position.

7. Ensemble selon la revendication 6, dans lequel le support d'aiguille de cartouche (83) est déplacé hors de l'agencement coaxial par rapport au curseur d'aiguille de cartouche (82) lorsque l'axe d'aiguille (53) n'est pas parallèle à l'axe d'insertion (52) lorsque le curseur d'aiguille de cartouche (82) est déplacé dans la seconde position, permettant ainsi de générer des forces de pénétration d'aiguille perpendiculaires à l'axe d'insertion (52) déformant l'accouplement souple.

8. Ensemble selon l'une quelconque des revendications précédentes, dans lequel le support d'aiguille de cartouche (83) est fixé axialement au curseur d'aiguille de cartouche (82) tout en permettant un mouvement par rapport au curseur d'aiguille de cartouche (82) perpendiculaire à l'axe d'insertion (52), ou un mouvement de pivotement ou de rotation autour de l'axe d'insertion (53) contre une sollicitation ou une résistance de l'accouplement souple et dans lequel le curseur d'aiguille de cartouche (82) est guidé linéairement par le boîtier (10, 11) par une mise en prise cannelée définissant l'axe d'insertion.

9. Ensemble selon l'une quelconque des revendications précédentes, dans lequel l'aiguille de cartouche (70) est reliée au support d'aiguille de cartouche (83) par un élément élastique (70a) qui est de préférence une colle élastique.

10. Ensemble selon l'une quelconque des revendications précédentes, dans lequel le dispositif d'entraînement est un ressort (90) situé entre le boîtier (10, 11) et le curseur d'aiguille de cartouche (82) et agissant sur le boîtier (10, 11) et le curseur d'aiguille de cartouche (83), le ressort (90) étant maintenu dans un état comprimé pour fournir une force de sollicitation sur le boîtier (10, 11) et le curseur d'aiguille de cartouche (82) lorsque le curseur d'aiguille de cartouche (82) est maintenu dans la première position par rapport au boîtier par un dispositif de verrouillage libérable.

11. Ensemble selon l'une quelconque des revendications précédentes, dans lequel le premier élément d'accouplement (83a, 83b) est creux et forme un passage pour un conduit de fluide établissant une liaison fluidique entre un médicament présent dans la cartouche (4) et l'aiguille de cartouche (70).

12. Ensemble selon l'une quelconque des revendications précédentes, dans lequel l'aiguille de cartouche (70) est une aiguille creuse, moyennant quoi la pointe de l'aiguille est aiguisée et ayant une extrémité fermée, telle qu'une aiguille à pointe de crayon, ou la pointe de l'aiguille est aiguisée et a une extrémité ouverte.

13. Dispositif d'injection comprenant l'ensemble selon l'une quelconque des revendications précédentes, dans lequel le dispositif d'injection est un dispositif d'injection de timbre prérempli comprenant une surface adhésive cutanée et une aiguille cutanée (25) conçue pour se déplacer d'une position rétractée d'aiguille dans le boîtier (10, 11) à une position d'insertion d'aiguille au moins partiellement à l'extérieur du boîtier après que le curseur d'aiguille de cartouche (82) s'est déplacé de la première position à la seconde position.
